(19) Europäisches Patentamt
European Patent Office
Office européen des brevets

(11) Veröffentlichungsnummer: **0 483 465 B1**

(12) **EUROPÄISCHE PATENTSCHRIFT**

(45) Veröffentlichungstag der Patentschrift: **02.08.95**

(51) Int. Cl.6: **C07F 7/18**, C07K 5/08, C07K 5/10, C07K 7/06, A61K 9/50, A61K 9/52, A61K 39/00

(21) Anmeldenummer: **91113199.3**

(22) Anmeldetag: **06.08.91**

# (54) Langkettige Di(acyloxy)dialkylsilane, Di(acyloxy)diarylsilane, Di(acyloxy)dialkoxysilane und Tetra(acyloxy)silane, Verfahren zu ihrer Herstellung, ihre Verwendung zur Herstellung von Vesikeln, aus ihnen hergestellte Vesikel (Siosomen) und deren Verwendung als träger von Wirkstoffen.

(30) Priorität: **07.08.90 DD 343282**

(43) Veröffentlichungstag der Anmeldung:
**06.05.92 Patentblatt 92/19**

(45) Bekanntmachung des Hinweises auf die Patenterteilung:
**02.08.95 Patentblatt 95/31**

(84) Benannte Vertragsstaaten:
**CH DE FR GB IT LI**

(56) Entgegenhaltungen:
**FR-A- 2 236 911**
**US-A- 4 007 314**

**CHEMICAL ABSTRACTS Band 87, Nr. 6, 8. August1977, Seite 39, Zusammenfassung Nr. 40370y, Columbus, Ohio, US; & JP- A - 7737964 (SHIN-ETSU CHEMICAL INDUSTRY CO. LTD) 24.03.1977**

(73) Patentinhaber: **Salama, Zoser Boulis, Dr.**
**Hudlerstrasse 50**
**D-89250 Senden (DE)**

Patentinhaber: **Richter, Heinrich, Doz. Dr. rer.nat. habil.**
**Rudolf-Heym-Strasse 18**
**D-06110 Halle, Saale (DE)**

Patentinhaber: **Meyer, Helmut-Walter, Prof. Dr. sc.**
**Naumburger Strasse 31**
**D-07743 Jena (DE)**

Patentinhaber: **Kunath, Ute, Dr.**
**Ernst-Moritz-Arndt-Strasse 3**
**D-06886 Lutherstadt Wittenberg (DE)**

Patentinhaber: **Strube, Michael, Dr.**
**An der eigenen Scholle 17**
**D-06130 Halle, Saale (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS Band 84, Nr. 10, 8. März1986, Seite 42, Zusammenfassung Nr. 60547c, Columbus, Ohio, US; &JP - A - 75123755 (SHIN-ETSU CHEMICAL INDUSTRY CO. LTD) 29.09.1975

CHEMICAL ABSTRACTS Band 77, Nr. 1, 3. Juli 1972,Seite 512, Zusammenfassung Nr. 5771b, Columbus, Ohio, US; H. BUCHWALD et al.:"Reaction of halosilanes with N-protected amino acids"& J. Organometal. Chem. 1972, Band 37, Nr. 1, Seiten C1-C3

CHEMICAL ABSTRACTS Band 108, Nr. 19, 9. Mai1988, Seite 79, Zusammenfassung Nr. 161596n, Columbus, Ohio, US; J. MILLERSHIPet al.: "Prodrugs utilizing organosilyl derivation: an investigation of thelong-term androgenic and myotropic activities of silyl derivatives oftestosterone" & J. Pharm. Sci. 1988, Band

77, Nr. 2, Seiten 116-119

CHEMICAL ABSTRACTS Band 108, Nr. 4, 25. Januar1988, Seite 105, Zusammenfassung Nr. 23732c, Columbus, Ohio, US; D. JAEGER etal.: "Preparation and characterization of double-chain destructible surfactantsand derived vesicles" & J. Am. Oil.Chem. Soc. 1987, Band 64, Nr. 11, Seiten 1550-1551

Patentinhaber: **Nuhn, Peter, Prof. Dr. sc.**
**Naunhofer Strasse 53**
**D-04299 Leipzig (DE)**

Patentinhaber: **Nindel, Horst, Dr.**
**Uranusstrasse 40**
**D-06118 Halle (DE)**

(72) Erfinder: **Salama, Zoser Boulis, Dr.**
**Hudlerstrasse 50**
**D-89250 Senden (DE)**
Erfinder: **Richter, Heinrich, Doz. Dr. rer.nat. habil.**
**Rudolf-Heym-Strasse 18**
**D-06110 Halle, Saale (DE)**
Erfinder: **Meyer, Helmut-Walter, Prof. Dr. sc.**
**Naumburger Strasse 31**
**D-07743 Jena (DE)**
Erfinder: **Kunath, Ute, Dr.**
**Ernst-Moritz-Arndt-Strasse 3**
**D-06886 Lutherstadt Wittenberg (DE)**
Erfinder: **Strube, Michael, Dr.**
**An der eigenen Scholle 17**
**D-06130 Halle, Saale (DE)**
Erfinder: **Nuhn, Peter, Prof. Dr. sc.**
**Naunhofer Strasse 53**
**D-04299 Leipzig (DE)**
Erfinder: **Nindel, Horst, Dr.**
**Uranusstrasse 40**
**D-06118 Halle (DE)**

## Beschreibung

Die Erfindung betrifft neue langkettige Di(acyloxy)dialkylsilane, Di(acyloxy)diarylsilane, Di(acyloxy)-dialkoxysilane und Tetra(acyloxy)silane, ein Verfahren zu ihrer Herstellung, ihre Verwendung zur Herstellung von Vesikeln, aus den langkettigen Di(acyloxy)dialkylsilanen, Di(acyloxy)diarylsilanen, Di(acyloxy)-dialkoxysilanen und Tetra(acyloxy)silanen, bestehende Vesikel (Siosomen) und deren Verwendung als Träger verschiedenartiger Wirkstoffe.

Bilayer-Vesikel aus Phospholipiden (Lecithinen), die konzentrische, in sich geschlossene Anordnungen aufweisen, sind unter dem Begriff Liposomen bekannt. Sie bestehen aus bimolekularen Schalen, die im Inneren, d.h. in ihrem Kern und zwischen den Schalen,wäßrige Kompartimente aufweisen. In diesen wäßrigen Kompartimenten können sich auch Substanzen befinden, die in dem Wasser vorher bereits gelöst waren, das bei der Herstellung der Liposomen verwendet wurde. Die Liposomen können also dazu dienen, Wirkstoffe einzuschließen und stellen Träger für diese Wirkstoffe dar. Die Wirkstoffe können niedermolekulare Stoffe sein, beispielsweise Arzneistoffe, oder auch hochmolekulare Stoffe, beispielsweise Proteine.

Die Liposomen auf Phospholipid-Basis weisen den Nachteil einer unzureichenden Stabilität gegen äußere Einflüsse, wie Temperaturerhöhung oder Licht auf.

Außerdem sind sie nicht leicht reproduzierbar in definierter chemischer Zusammensetzung herzustellen (Arndt, D.; Fichtner, I.: Liposomen, Darstellung - Eigenschaften - Anwendung in Fortschritte der Onkologie. Akademie-Verlag, Berlin 1986 Band 13 S.1-141).

Verbindungen der allgemeinen Formel I werden bisher weder in der Patent- noch in der Fachliteratur beschrieben. Bekannt sind lediglich Verfahren zur kontinuierlichen Veresterung von Chlorsilanen, wobei als Ausgangssubstanz immer Trichlorsilan bzw. Vinyltrichlorsilan verwendet werden und die Alkylreste nur Kettenlängen bis zu 4 Kohlenstoffatomen aufweisen. Die Veresterungen erfolgen dabei immer mit entsprechenden Alkoholen.

DDR C 07 F 7/02 120024
DDR C 07 F 7/02 106388
DDR C 07 F 7/04 118432
DDR C 07 F 7/04 79473
DDR C 07 F 7/08 210281
DDR C 07 F 7/18 125707
DDR C 07 F 7/18 4173576
DDR C 07 F 7/18 2532887

Die von uns aus den Di(acyloxy)dialkylsilanen, Di(acyloxy)diarylsilanen, Di(acyloxy)dialkoxysilanen und Tetra(acyloxy)silanen hergestellten geordneten Vesikel (Siosomen) sind bisher weder in der Patent- noch in der Fachliteratur beschrieben.

Vesikel aus Organo-Silizium-Verbindungen sind bisher weder bekannt, noch hergestellt, noch beschrieben worden.

Der Erfindung liegt die Aufgabe zugrunde, neue chemische Verbindungen bereitzustellen, die sich zur Herstellung von Nicht-Phospholipid-Vesikeln eignen. Eine weitere Aufgabe der Erfindung besteht in der Bereitstellung neuer Nicht-Phospholipid-Vesikel, die chemisch stabil, gegen Einflüsse wie Temperaturerhöhung oder Lichteinwirkung unempfindlich und mit definierter Zusammensetzung herstellbar sind. Diese Aufgaben werden durch die Erfindung gelöst.

Gegenstand der Erfindung sind somit neue langkettige Di(acyloxy)dialkylsilane, Di(acyloxy)diarylsilane, Di(acyloxy)dialkoxysilane und Tetra(acyloxy)silane, der allgemeinen Formel I

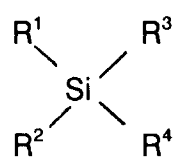

in der $R^1$ und $R^2$, die gleich oder verschieden sein können, jeweils einen Acyloxyrest der Formel R-COO- oder einen Peptidrest der Formel

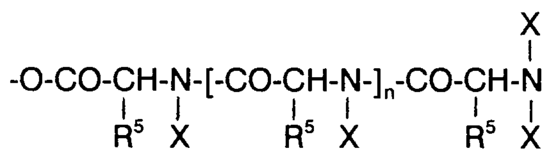

bedeuten, wobei R einen unverzweigten oder verzweigten Alkyl-, Alkenyl- oder Alkinylrest mit 5 bis 29 C-Atomen bedeutet, die durch ein bis drei Halogenatome, Alkoxyreste mit 1 bis 18 C-Atomen oder Aminogruppen substituiert sein können, die Reste $R^5$, die gleich oder verschieden sein können, den nach der Entfernung der Gruppe

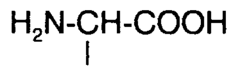

von einer in der Natur vorkommenden Aminosäure verbleibenden Rest darstellen, die Reste X, die gleich oder verschieden sein können, ein Wasserstoffatom oder eine in der Peptidchemie übliche Aminoschutzgruppe darstellen und n eine ganze Zahl von 1 bis 12 bedeutet, und $R^3$ und $R^4$, die gleich oder verschieden sein können, jeweils einen Alkylrest mit 1 bis 6 C-Atomen, eine Arylgruppe mit 3 bis 14 C-Atomen, beispielsweise eine Phenylgruppe, den nach Entfernung eines Wasserstoffatoms verbleibenden Rest eines Monosaccharids, Disaccharids, Aminozuckers oder einer Hydroxycarbonsäure, einen Alkoxyrest mit 1 bis 5 C-Atomen, einen Acylrest, einer in der Natur vorkommenden Aminosäure mit freier oder geschützter Aminogruppe oder einen Di-, Tri- oder Tetrapeptidrest aus einer in der Natur vorkommenden Aminosäure mit freien oder geschützten Aminogruppen darstellen oder die Bedeutung $R^1$ und $R^2$ haben, wobei R einen unverzweigten oder verzweigten Alkyl-, Alkenyl-, oder Alkinylrest darstellt.

Gegenstand der Erfindung ist ferner die Verwendung der Verbindungen der allgemeinen Formel I zur Herstellung von Vesikeln auf Nicht-Phospholipid-Basis.

Gegenstand der Erfindung sind ferner Vesikel auf Nicht-Phospholipid-Basis, die aus mindestens einer konzentrischen, in sich geschlossenen Schicht von langkettigen Di(acyloxy)dialkylsilanen, Di(acyloxy)-diarylsilanen, Di(acyloxy)dialkoxysilanen und Tetra(acyloxy)silanen, der allgemeinen Formel I bestehen. Die Vesikel der Erfindung werden als "Siosomen" bezeichnet. Sie können in ihren Kompartimenten - ebenso wie die bekannten Liposomen - eine Vielzahl von Wirkstoffen eingeschlossen enthalten.

Gegenstand der Erfindung ist deshalb schließlich auch die Verwendung der Siosomen-Vesikel als Träger von Wirkstoffen, wie Pharmazeutika, Kosmetika, Lebens-und Futtermitteln, Enzymen, Vitaminen und Agrochemikalien.

Die Verbindungen der allgemeinen Formel I enthalten zwei an das Si-Atom gebundene langkettige und zwei ebenfalls an das Si-Atom gebundene verhältnismaßig kurzkettige Reste. Die langkettigen, in der Formel I mit $R^1$ und $R^2$ bezeichneten Reste können Acyloxyreste der Formel R-COO- oder Peptidreste der Formel

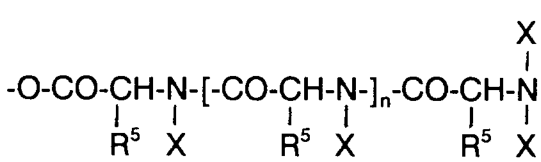

sein.

Die Acyloxyreste der Formel R-COO- weisen 6 bis 30 C-Atome auf, d.h. die Reste R enthalten 5 bis 29 C-Atome. Bevorzugt sind Alkyl-, Alkenyl- und Alkinylreste mit 6 bis 18 C-Atomen.

Die Reste R können 1 bis 3 Substituenten tragen, nämlich Halogenatome, Alkoxyreste mit 1 bis 18 C-Atomen oder Aminogruppen. Die Reste R selbst können unverzweigt oder verzweigt sein.

In einer zweiten Ausführungsform bedeuten die Reste $R^1$ und $R^2$ Peptidreste der vorstehend angegebenen Formel. In dieser Formel ist der Rest

$$\text{-CO-}\underset{\substack{|\\ R^5}}{\text{CH}}\text{-N-}$$

der Rest einer in der Natur vorkommenden Aminosäure der allgemeinen Formel

$$H_2N\text{-}\underset{\substack{|\\ R^5}}{\text{CH}}\text{-COOH},$$

also beispielsweise von Isoleucin, Leucin, Lysin, Methionin, Phenylalanin, Threonin, Tryptophan, Valin, Alanin, Arginin, Asparagin, Cystein, Glutamin, Glutaminsäure, Glycin, Hydroxylysin, Hydroxyprolin, Prolin, Serin oder Thyrosin.

Die Peptidreste der vorstehend angegebenen Formel in der Bedeutung von $R^1$ und $R^2$ können Homopolymere sein, d.h. alle Reste $R^5$ haben die gleiche Bedeutung, oder Copolymere, bei denen die Reste $R^5$ unterschiedliche Bedeutung haben, oder Sequenzcopolymere, in denen die aufeinanderfolgenden Aminosäureeinheiten regelmäßig angeordnet sind.

In den Peptidresten der vorstehend angegebenen Formel können die Aminogruppen in freier Form vorliegen, d.h. X hat die Bedeutung Wasserstoffatom, oder sie können die auf dem Gebiet der Peptidchemie üblichen Schutzgruppen tragen, beispielsweise Alkyl- oder Acylreste.

Die verhältnismäßig kurzkettigen Reste $R^3$ und $R^4$ in der Formel I können in einer Ausführungsform Alkylreste mit 1 bis 6, vorzugsweise 1 bis 4 C-Atomen oder Arylgruppen mit 3 bis 14 C-Atomen, vorzugsweise Phenylgruppen sein. In einer weiteren Ausführungsform können diese Reste die nach Entfernung eines Wasserstoffatoms verbleibenden Reste eines Monosaccharids, Disaccharids, Aminozukkers oder einer Hydroxycarbonsäure sein.

Spezielle Beispiele für geeignete Monosaccharide sind Pentosen, wie Arabinose, Ribose und Xylose, sowie Hexosen, wie Glucose, Mannose, Galactose und Fructose. Beispiele für geeignete Disaccharide sind Saccharose, Lactose und Maltose. Beispiele für geeignete Aminozucker sind Glucosamin und Galactosamin. Ein Beipiel für eine geeignete Hydroxycarbonsäure ist Glucuronsäure. Die Hydroxygruppen der Hydroxycarbonsäuren können dabei in freier, teilderivatisierter oder vollständig derivatisierter Form (Schutzgruppen) vorliegen.

Spezielle Beispiele für langkettige Di(acyloxy)dialkylsilane, Di(acyloxy)diarylsilane, Di(acyloxy)-dialkoxysilane und Tetra(acyloxy)silane der Erfindung sind in der nachstehenden Auflistung angegeben.

| Name | Summenformel | Molekulargewicht |
|---|---|---|
| * Di(octanoyloxy)dimethylsilan | $C_{18}H_{36}O_4Si$ | (344) |
| * Di(decanoyloxy)dimethylsilan | $C_{22}H_{44}O_4Si$ | (400) |
| * Di(dodecanoyloxy)dimethylsilan | $C_{26}H_{52}O_4Si$ | (456) |
| * Di(tetradecanoyloxy)dimethylsilan | $C_{30}H_{60}O_4Si$ | (512) |
| * Di(hexadecanoyloxy)dimethylsilan | $C_{34}H_{68}O_4Si$ | (568) |
| * Di(octadecanoyloxy)dimethylsilan | $C_{38}H_{76}O_4Si$ | (624) |
| Di(nonanoyloxy)dimethylsilan | $C_{20}H_{40}O_4Si$ | (372) |
| Di(undecanoyloxy)dimethylsilan | $C_{24}H_{48}O_4Si$ | (428) |
| Di(tridecanoyloxy)dimethylsilan | $C_{28}H_{56}O_4Si$ | (484) |
| Di(pentadecanoyloxy)dimethylsilan | $C_{32}H_{64}O_4Si$ | (540) |
| Di(heptadecanoyloxy)dimethylsilan | $C_{36}H_{72}O_4Si$ | (596) |
| Di(octanoyloxy)diethylsilan | $C_{20}H_{40}O_4Si$ | (372) |
| Di(nonanoyloxy)diethylsilan | $C_{22}H_{44}O_4Si$ | (400) |
| Di(decanoyloxy)diethylsilan | $C_{24}H_{48}O_4Si$ | (428) |
| Di(undecanoyloxy)diethylsilan | $C_{26}H_{52}O_4Si$ | (456) |
| Di(dodecanoyloxy)diethylsilan | $C_{28}H_{56}O_4Si$ | (484) |
| Di(tridecanoyloxy)diethylsilan | $C_{30}H_{60}O_4Si$ | (512) |
| Di(tetradecanoyloxy)diethylsilan | $C_{32}H_{64}O_4Si$ | (540) |
| Di(pentadecanoyloxy)diethylsilan | $C_{34}H_{68}O_4Si$ | (568) |
| Di(hexadecanoyloxy)diethylsilan | $C_{36}H_{72}O_4Si$ | (596) |
| Di(heptadecanoyloxy)diethylsilan | $C_{38}H_{76}O_4Si$ | (624) |
| Di(octadecanoyloxy)diethylsilan | $C_{40}H_{80}O_4Si$ | (652) |
| Di(octanoyloxy)dipropylsilan | $C_{22}H_{44}O_4Si$ | (400) |

| | | |
|---|---|---|
| Di(nonanoyloxy)dipropylsilan | $C_{24}H_{48}O_4Si$ | (428) |
| Di(decanoyloxy)dipropylsilan | $C_{26}H_{52}O_4Si$ | (456) |
| Di(undecanoyloxy)dipropylsilan | $C_{28}H_{56}O_4Si$ | (484) |
| Di(dodecanoyloxy)dipropylsilan | $C_{30}H_{60}O_4Si$ | (512) |
| Di(tridecanoyloxy)dipropylsilan | $C_{32}H_{64}O_4Si$ | (540) |
| Di(tetradecanoyloxy)dipropylsilan | $C_{34}H_{68}O_4Si$ | (568) |
| Di(pentadecanoyloxy)dipropylsilan | $C_{36}H_{72}O_4Si$ | (596) |
| Di(hexadecanoyloxy)dipropylsilan | $C_{38}H_{76}O_4Si$ | (624) |
| Di(heptadecanoyloxy)dipropylsilan | $C_{40}H_{80}O_4Si$ | (652) |
| Di(octadecanoyloxy)dipropylsilan | $C_{42}H_{84}O_4Si$ | (680) |
| Di(octanoyloxy)diisopropylsilan | $C_{22}H_{44}O_4Si$ | (400) |
| Di(nonanoyloxy)diisopropylsilan | $C_{24}H_{48}O_4Si$ | (428) |
| Di(decanoyloxy)diisopropylsilan | $C_{26}H_{52}O_4Si$ | (456) |
| Di(undecanoyloxy)diisopropylsilan | $C_{28}H_{56}O_4Si$ | (484) |
| Di(dodecanoyloxy)diisopropylsilan | $C_{30}H_{60}O_4Si$ | (512) |
| Di(tridecanoyloxy)diisopropylsilan | $C_{32}H_{64}O_4Si$ | (540) |
| Di(tetradecanoyloxy)diisopropylsilan | $C_{34}H_{68}O_4Si$ | (568) |
| Di(pentadecanoyloxy)diisopropylsilan | $C_{36}H_{72}O_4Si$ | (596) |
| Di(hexadeccanoyloxy)diisopropylsilan | $C_{38}H_{76}O_4Si$ | (624) |
| Di(heptadecanoyloxy)diisopropylsilan | $C_{40}H_{80}O_4Si$ | (652) |
| Di(octadecanoyloxy)diisopropylsilan | $C_{42}H_{84}O_4Si$ | (680) |
| Di(linoylooxy)dimethylsilan | $C_{38}H_{68}O_4Si$ | (616) |
| Di(linolenoylooxy)dimethylsilan | $C_{38}H_{64}O_4Si$ | (612) |
| Di(oleylooxy)dimethylsilan | $C_{38}H_{72}O_4Si$ | (620) |
| Di(linoylooxy)diethylsilan | $C_{40}H_{72}O_4Si$ | (644) |
| Di(linolenoylooxy)diethylsilan | $C_{40}H_{68}O_4Si$ | (640) |
| Di(oleylooxy)diethylsilan | $C_{40}H_{76}O_4Si$ | (648) |
| Di(linoylooxy)dipropylsilan | $C_{42}H_{76}O_4Si$ | (672) |
| Di(linolenoylooxy)dipropylsilan | $C_{42}H_{72}O_4Si$ | (668) |

| | | |
|---|---|---|
| Di(oleylooxy)dipropylsilan | $C_{42}H_{80}O_4Si$ | (676) |
| Di(linoylooxy)diisopropylsilan | $C_{42}H_{76}O_4Si$ | (672) |
| Di(linolenoylooxy)diisopropylsilan | $C_{42}H_{72}O_4Si$ | (668) |
| Di(oleylooxy)diisopropylsilan | $C_{42}H_{80}O_4Si$ | (676) |
| Dimethyl bis(triglycyl)silylester | $C_{14}H_{26}O_8N_6Si$ | (434) |
| Dimethyl bis(tetraglycyl)silylester | $C_{18}H_{32}O_{10}N_8Si$ | (548) |
| Dimethyl bis(pentaglycyl)silylester | $C_{22}H_{38}O_{12}N_{10}Si$ | (662) |
| Dimethyl bis(hexaglycyl)silylester | $C_{26}H_{44}O_{14}N_{12}Si$ | (776) |
| Dimethyl bis(heptaglycyl)silylester | $C_{30}H_{50}O_{16}N_{14}Si$ | (890) |
| Dimethyl bis(octaglycyl)silylester | $C_{34}H_{56}O_{18}N_{16}Si$ | (1004) |
| Dimethyl bis(nonaglycyl)silylester | $C_{38}H_{62}O_{20}N_{18}Si$ | (1118) |
| Dimethyl bis(decaglycyl)silylester | $C_{42}H_{68}O_{22}N_{20}Si$ | (1232) |
| Diethyl bis(triglycyl)silylester | $C_{16}H_{30}O_8N_6Si$ | (462) |
| Diethyl bis(tetraglycyl)silylester | $C_{20}H_{36}O_{10}N_8Si$ | (576) |
| Diethyl bis(pentaglycyl)silylester | $C_{24}H_{42}O_{12}N_{10}Si$ | (690) |
| Diethyl bis(hexaglycyl)silylester | $C_{28}H_{48}O_{14}N_{12}Si$ | (804) |
| Diethyl bis(heptaglycyl)silylester | $C_{32}H_{54}O_{16}N_{14}Si$ | (918) |
| Diethyl bis(octablycyl)silylester | $C_{36}H_{60}O_{18}N_{16}Si$ | (1032) |
| Diethyl bis(nonaglycyl)silylester | $C_{40}H_{66}O_{20}N_{18}Si$ | (1146) |
| Diethyl bis(decaglycyl)silylester | $C_{44}H_{72}O_{22}N_{20}Si$ | (1260) |
| Dipropyl bis(triglycyl)silylester | $C_{18}H_{34}O_8N_6Si$ | (490) |
| Dipropyl bis(tetraglycyl)silylester | $C_{22}H_{40}O_{10}N_8Si$ | (604) |
| Dipropyl bis(pentaglycyl)silylester | $C_{26}H_{46}O_{12}N_{10}Si$ | (718) |
| Dipropyl bis(hexaglycyl)silylester | $C_{30}H_{52}O_{14}N_{12}Si$ | (832) |
| Dipropyl bis(heptaglycyl)silylester | $C_{34}H_{58}O_{16}N_{14}Si$ | (946) |
| Dipropyl bis(octaglycyl)silylester | $C_{38}H_{64}O_{18}N_{16}Si$ | (1060) |
| Dipropyl bis(nonaglycyl)silylester | $C_{42}H_{70}O_{20}N_{18}Si$ | (1174) |
| Dipropyl bis(decaglycyl)silylester | $C_{46}H_{76}O_{22}N_{20}Si$ | (1288) |
| Diisopropyl bis(triglycyl)silylester | $C_{18}H_{34}O_8N_6Si$ | (490) |

| | | |
|---|---|---|
| Diisopropyl bis(tetraglycyl)silylester | $C_{22}H_{40}O_{10}N_8Si$ | (604) |
| Diisopropyl bis(pentaglycyl)silylester | $C_{26}H_{46}O_{12}N_{10}Si$ | (718) |
| Diisopropyl bis(hexaglycyl)silylester | $C_{30}H_{52}O_{14}N_{12}Si$ | (832) |
| Diisopropyl bis(heptaglycyl)silylester | $C_{34}H_{58}O_{16}N_{14}Si$ | (946) |
| Diisopropyl bis(octaglycyl)silylester | $C_{38}H_{64}O_{18}N_{16}Si$ | (1060) |
| Diisopropyl bis(nonaglycyl)silylester | $C_{42}H_{70}O_{20}N_{18}Si$ | (1174) |
| Diisopropyl bis(decaglycyl)silylester | $C_{46}H_{76}O_{22}N_{20}Si$ | (1288) |
| Dimethyl bis(trialanyl)silylester | $C_{20}H_{38}O_8N_6Si$ | (518) |
| Dimethyl bis(tetraalanyl)silylester | $C_{26}H_{48}O_{10}N_8Si$ | (660) |
| Dimethyl bis(pentaalanyl)silylester | $C_{32}H_{58}O_{12}N_{10}Si$ | (802) |
| Dimethyl bis(hexaalanyl)silylester | $C_{38}H_{68}O_{14}N_{12}Si$ | (944) |
| Dimethyl bis(heptaalanyl)silylester | $C_{44}H_{78}O_{16}N_{14}Si$ | (1086) |
| Dimethyl bis(octaalanyl)silylester | $C_{50}H_{88}O_{18}N_{16}Si$ | (1228) |
| Dimethyl bis(nonaalanyl)silylester | $C_{56}H_{98}O_{20}N_{18}Si$ | (1370) |
| Dimethyl bis(decaalanyl)silylester | $C_{62}H_{108}O_{22}N_{20}Si$ | (1512) |
| Diethyl bis(trialanyl)silylester | $C_{22}H_{42}O_8N_6Si$ | (546) |
| Diethyl bis(tetraalanyl)silylester | $C_{28}H_{52}O_{10}N_8Si$ | (688) |
| Diethyl bis(pentaalanyl)silylester | $C_{34}H_{62}O_{12}N_{10}Si$ | (830) |
| Diethyl bis(hexaalanyl)silylester | $C_{40}H_{72}O_{14}N_{12}Si$ | (972) |
| Diethyl bis(heptaalanyl)silylester | $C_{46}H_{82}O_{16}N_{14}Si$ | (1114) |
| Diethyl bis(octaalanyl)silylester | $C_{52}H_{92}O_{18}N_{16}Si$ | (1256) |
| Diethyl bis(nonaalanyl)silylester | $C_{58}H_{102}O_{20}N_{18}Si$ | (1398) |
| Diethyl bis(decaalanyl)silylester | $C_{64}H_{112}O_{22}N_{20}Si$ | (1540) |
| Dipropyl bis(trialanyl)silylester | $C_{24}H_{46}O_8N_6Si$ | (574) |
| Dipropyl bis(tetraalanyl)silylester | $C_{30}H_{56}O_{10}N_8Si$ | (716) |
| Dipropyl bis(pentaalanyl)silylester | $C_{36}H_{66}O_{12}N_{10}Si$ | (858) |
| Dipropyl bis(hexaalanyl)silylester | $C_{42}H_{76}O_{14}N_{12}Si$ | (1000) |
| Dipropyl bis(heptaalanyl)silylester | $C_{48}H_{86}O_{16}N_{14}Si$ | (1142) |
| Dipropyl bis(octaalanyl)silylester | $C_{54}H_{96}O_{18}N_{16}Si$ | (1284) |

| | | |
|---|---|---|
| Dipropyl bis(nonaalanyl)silylester | $C_{60}H_{106}O_{20}N_{18}Si$ | (1426) |
| Dipropyl bis(decaalanyl)silylester | $C_{66}H_{116}O_{22}N_{20}Si$ | (1568) |
| Diisopropyl bis(trialanyl)silylester | $C_{24}H_{46}O_{8}N_{6}Si$ | (574) |
| Diisopropyl bis(tetraalanyl)silylester | $C_{30}H_{56}O_{10}N_{8}Si$ | (716) |
| Diisopropyl bis(pentaalanyl)silylester | $C_{36}H_{66}O_{12}N_{10}Si$ | (858) |
| Diisopropyl bis(hexaalanyl)silylester | $C_{42}H_{76}O_{14}N_{12}Si$ | (1000) |
| Diisopropyl bis(heptaalanyl)silylester | $C_{48}H_{86}O_{16}N_{14}Si$ | (1142) |
| Diisopropyl bis(octaalanyl)silylester | $C_{54}H_{96}O_{18}N_{16}Si$ | (1284) |
| Diisopropyl bis(nonaalanyl)silylester | $C_{60}H_{106}O_{20}N_{18}Si$ | (1426) |
| Diisopropyl bis(decaalanyl)silylester | $C_{66}H_{116}O_{22}N_{20}Si$ | (1568) |
| Dimethyl bis(trivalyl)silylester | $C_{32}H_{62}O_{8}N_{6}Si$ | (686) |
| Dimethyl bis(tetravalyl)silylester | $C_{42}H_{80}O_{10}N_{8}Si$ | (884) |
| Dimethyl bis(pentavalyl)silylester | $C_{52}H_{98}O_{12}N_{10}Si$ | (1082) |
| Dimethyl bis(hexavalyl)silylester | $C_{62}H_{116}O_{14}N_{12}Si$ | (1280) |
| Dimethyl bis(heptavalyl)silylester | $C_{72}H_{134}O_{16}N_{14}Si$ | (1478) |
| Dimethyl bis(octavalyl)silylester | $C_{82}H_{152}O_{18}N_{16}Si$ | (1676) |
| Dimethyl bis(nonavalyl)silylester | $C_{92}H_{170}O_{20}N_{18}Si$ | (1874) |
| Dimethyl bis(decavalyl)silylester | $C_{102}H_{188}O_{22}N_{20}Si$ | (2072) |
| Diethyl bis(trivalyl)silylester | $C_{34}H_{66}O_{8}N_{6}Si$ | (714) |
| Diethyl bis(tetravalyl)silylester | $C_{44}H_{84}O_{10}N_{8}Si$ | (912) |
| Diethyl bis(pentavalyl)silylester | $C_{54}H_{102}O_{12}N_{10}Si$ | (1110) |
| Diethyl bis(hexavalyl)silylester | $C_{64}H_{120}O_{14}N_{12}Si$ | (1308) |
| Diethyl bis(heptavalyl)silylester | $C_{74}H_{138}O_{16}N_{14}Si$ | (1506) |
| Diethyl bis(octavalyl)silylester | $C_{84}H_{156}O_{18}N_{16}Si$ | (1704) |
| Diethyl bis(nonavalyl)silylester | $C_{94}H_{174}O_{20}N_{18}Si$ | (1902) |
| Diethyl bis(decavalyl)silylester | $C_{104}H_{192}O_{22}N_{20}Si$ | (2100) |
| Dipropyl bis(trivalyl)silylester | $C_{36}H_{70}O_{8}N_{6}Si$ | (742) |
| Dipropyl bis(tetravalyl)silylester | $C_{46}H_{88}O_{10}N_{8}Si$ | (940) |
| Dipropyl bis(pentavalyl)silylester | $C_{56}H_{106}O_{12}N_{10}Si$ | (1138) |

| | | |
|---|---|---|
| Dipropyl bis(hexavalyl)silylester | $C_{66}H_{124}O_{14}N_{12}Si$ | (1336) |
| Dipropyl bis(heptavalyl)silylester | $C_{76}H_{142}O_{16}N_{14}Si$ | (1534) |
| Dipropyl bis(octavalyl)silylester | $C_{86}H_{160}O_{18}N_{16}Si$ | (1732) |
| Dipropyl bis(nonavalyl)silylester | $C_{96}H_{178}O_{20}N_{18}Si$ | (1930) |
| Dipropyl bis(decavalyl)silylester | $C_{106}H_{196}O_{22}N_{20}Si$ | (2128) |
| Dimethyl bis(triphenylalanyl)silylester | $C_{56}H_{62}O_{8}N_{6}Si$ | (974) |
| Dimethyl bis(tetraphenylalanyl)silylester | $C_{74}H_{80}O_{10}N_{8}Si$ | (1268) |
| Dimethyl bis(pentaphenylalanyl)silylester | $C_{92}H_{98}O_{12}N_{10}Si$ | (1562) |
| Dimethyl bis(hexaphenylalanyl)silylester | $C_{110}H_{116}O_{14}N_{12}Si$ | (1856) |
| Dimethyl bis(heptaphenylalanyl)silylester | $C_{128}H_{134}O_{16}N_{14}Si$ | (2150) |
| Dimethyl bis(octaphenylalanyl)silylester | $C_{146}H_{152}O_{18}N16Si$ | (2444) |
| Dimethyl bis(nonaphenylalanyl)silylester | $C_{164}H_{170}O_{20}N_{18}Si$ | (2738) |
| Dimethyl bis(decaphenylalanyl)silylester | $C_{182}H_{188}O_{22}N_{20}Si$ | (3032) |
| Diethyl bis(triphenylalanyl)silylester | $C_{58}H_{66}O_{8}N_{6}Si$ | (1002) |
| Diethyl bis(tetraphenylalnyl)silylester | $C_{76}H_{84}O_{10}N_{8}Si$ | (1296) |
| Diethyl bis(pentaphenylalanyl)silylester | $C_{94}H_{102}O_{12}N_{10}Si$ | (1590) |
| Diethyl bis(hexaphenylalanyl)silylester | $C_{112}H_{120}O_{14}N_{12}Si$ | (1884) |
| Diethyl bis(heptaphenylalanyl)silylester | $C_{130}H_{138}O_{16}N_{14}Si$ | (2178) |
| Diethyl bis(octaphenylalanyl)silylester | $C_{148}H_{156}O_{18}N_{16}Si$ | (2472) |
| Diethyl bis(nonaphenylalanyl)silylester | $C_{166}H_{174}O_{20}N_{18}Si$ | (2766) |
| Diethyl bis(decaphenylalanyl)silylester | $C_{184}H_{192}O_{22}N_{20}Si$ | (3060) |
| Dipropyl bis(triphenylalanyl)silylester | $C_{60}H_{70}O_{8}N_{6}Si$ | (1030) |
| Dipropyl bis(tetraphenylalanyl)silylester | $C_{78}H_{88}O_{10}N_{8}Si$ | (1324) |
| Dipropyl bis(pentaphenylalanyl)silylester | $C_{96}H_{106}O_{12}N_{10}Si$ | (1618) |
| Dipropyl bis(hexaphenylalanyl)silylester | $C_{114}H_{124}O_{14}N_{12}Si$ | (1912) |
| Dipropyl bis(heptaphenylalanyl)silylester | $C_{132}H_{142}O_{16}N_{16}Si$ | (2206) |
| Dipropyl bis(octaphenylalanyl)silylester | $C_{150}H_{160}O_{18}N_{16}Si$ | (2500) |
| Dipropyl bis(nonaphenylalanyl)silylester | $C_{168}H_{178}O_{20}N_{18}Si$ | (2794) |
| Dipropyl bis(decaphenylalanyl)silylester | $C_{186}H_{196}O_{22}N_{20}Si$ | (3088) |

| | | |
|---|---|---|
| * Di(octanoyloxy)diphenylsilan | $C_{28}H_{40}O_4Si$ | (468) |
| * Di(decanoyloxy)diphenylsilan | $C_{32}H_{48}O_4Si$ | (524) |
| * Di(dodecanoyloxy)diphenylsilan | $C_{36}H_{56}O_4Si$ | (581) |
| * Di(tetradecanoyloxy)diphenylsilan | $C_{40}H_{64}O_4Si$ | (637) |
| * Di(hexadecanoyloxy)diphenylsilan | $C_{44}H_{72}O_4Si$ | (693) |
| * Di(octadecanoyloxy)diphenylsilan | $C_{48}H_{80}O_4Si$ | (749) |
| Di(nonanoyloxy)diphenylsilan | $C_{30}H_{44}O_4Si$ | (496) |
| Di(undecanoyloxy)diphenylsilan | $C_{34}H_{52}O_4Si$ | (552) |
| Di(tridecanoyloxy)diphenylsilan | $C_{38}H_{60}O_4Si$ | (608) |
| Di(pentadecanoyloxy)diphenylsilan | $C_{42}H_{68}O_4Si$ | (664) |
| Di(heptadecanoyloxy)diphenylsilan | $C_{46}H_{76}O_4Si$ | (720) |
| Di(linoylooxy)diphenylsilan | $C_{48}H_{72}O_4Si$ | (740) |
| Di(linolenoylooxy)diphenylsilan | $C_{48}H_{68}O_4Si$ | (736) |
| Di(oleylooxy)diphenylsilan | $C_{48}H_{76}O_4Si$ | (744) |
| Diphenyl bis(triglycyl)silylester | $C_{24}H_{30}O_8N_6Si$ | (558) |
| Diphenyl bis(tetraglycyl)silylester | $C_{28}H_{36}O_{10}N_8Si$ | (672) |
| Diphenyl bis(pentaglycyl)silylester | $C_{32}H_{42}O_{12}N_{10}Si$ | (786) |
| Diphenyl bis(hexaglycyl)silylester | $C_{36}H_{48}O_{14}N_{12}Si$ | (900) |
| Diphenyl bis(heptaglycyl)silylester | $C_{40}H_{54}O_{16}N_{14}Si$ | (1014) |
| Diphenyl bis(octaglycyl)silylester | $C_{44}H_{60}O_{18}N_{16}Si$ | (1128) |
| Diphenyl bis(nonaglycyl)silylester | $C_{48}H_{66}O_{20}N_{18}Si$ | (1242) |
| Diphenyl bis(decaglycyl)silylester | $C_{52}H_{72}O_{22}N_{20}Si$ | (1356) |
| Diphenyl bis(trialanyl)silylester | $C_{30}H_{42}O_8N_6Si$ | (642) |
| Diphenyl bis(tetraalanyl)silylester | $C_{34}H_{48}O_{10}N_8Si$ | (756) |
| Diphenyl bis(pentaalanyl)silylester | $C_{38}H_{54}O_{12}N_{10}Si$ | (870) |
| Diphenyl bis(hexaalanyl)silylester | $C_{42}H_{60}O_{14}N_{12}Si$ | (984) |
| Diphenyl bis(heptaalanyl)silylester | $C_{46}H_{66}O_{16}N_{14}Si$ | (1098) |
| Diphenyl bis(octaalanyl)silylester | $C_{50}H_{72}O_{18}N_{16}Si$ | (1212) |
| Diphenyl bis(nonaalanyl)silylester | $C_{54}H_{78}O_{20}N_{18}Si$ | (1326) |

| | | |
|---|---|---|
| Diphenyl bis(decaalanyl)silylester | $C_{58}H_{84}O_{22}N_{20}Si$ | (1440) |
| Diphenyl bis(trivalyl)silylester | $C_{42}H_{66}O_8N_6Si$ | (810) |
| Diphenyl bis(tetravalyl)silylester | $C_{46}H_{72}O_{10}N_8Si$ | (924) |
| Diphenyl bis(pentavalyl)silylester | $C_{50}H_{78}O_{12}N_{10}Si$ | (1038) |
| Diphenyl bis(hexavalyl)silylester | $C_{54}H_{84}O_{14}N_{12}Si$ | (1152) |
| Diphenyl bis(heptavalyl)silylester | $C_{58}H_{90}O_{16}N_{14}Si$ | (1266) |
| Diphenyl bis(octavalyl)silylester | $C_{62}H_{96}O_{18}N_{16}Si$ | (1380) |
| Diphenyl bis(nonavalyl)silylester | $C_{66}H_{102}O_{20}N_{18}Si$ | (1494) |
| Diphenyl bis(decavalyl)silylester | $C_{70}H_{108}O_{22}N_{20}Si$ | (1608) |
| Diphenyl bis(triphenylalanyl)silylester | $C_{94}H_{90}O_8N_6Si$ | (1458) |
| Diphenyl bis(tetraphenylalanyl)silylester | $C_{98}H_{96}O_{10}N_8Si$ | (1572) |
| Diphenyl bis(pentaphenylalanyl)silylester | $C_{102}H_{102}O_{12}N_{10}Si$ | (1686) |
| Diphenyl bis(hexaphenylalanyl)silylester | $C_{106}H_{108}O_{14}N_{12}Si$ | (1800) |
| Diphenyl bis(heptaphenylalanyl)silylester | $C_{110}H_{114}O_{16}N_{14}Si$ | (1914) |
| Diphenyl bis(octaphenylalanyl)silylester | $C_{114}H_{120}O_{18}N_{16}Si$ | (2028) |
| Diphenyl bis(nonaphenylalanyl)silylester | $C_{118}H_{126}O_{20}N_{18}Si$ | (2142) |
| Diphenyl bis(decaphenylalanyl)silylester | $C_{122}H_{132}O_{22}N_{20}Si$ | (2256) |

* Angaben über Schmelzpunkt und Ausbeute sind in den Ausführungsbeispielen enthalten

Zur Herstellung der langkettigen Di(acyloxy)dialkylsilane, Di(acyloxy)diarylsilane, Di(acyloxy)-dialkoxysilane und Tetra(acyloxy)silane der Erfindung kann beispielsweise ein Dichlorsilan der allgemeinen Formel II

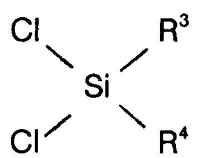

(II)

in der $R^3$ und $R^4$ die vorstehend angegebenen Bedeuteungen haben, mit einer Carbonsäure der Formel $R^1H$ bzw. $R^2H$ oder einem reaktiven Derivat davon, umgesetzt werden. $R^1$ und $R^2$ haben dabei die vorstehend angegebenen Bedeutungen. Beispielsweise kann man die Dichlorsilane (II) in wasserfreiem Ether mit den Natriumsalzen entsprechender Carbonsäuren mit den Acyloxyresten oder Peptidketten umsetzen. Die Umsetzung verläuft unter Rühren innerhalb einiger Stunden.

Zur Aufarbeitung wird zunächst überschüssiges Dichlorsilan durch Hinzufügen von Wasser hydrolysiert. Danach wird mehrmals mit Ether oder einem anderen Lösungsmittel extrahiert, die vereinigten Ertrakte gesammelt, getrocknet und im Vakuum eingeengt. Die erhaltenen Silane der Erfindung können dann aus einem geeigneten Lösungsmittel umkristallisiert werden.

Die disubstituierten Diphenylsilane als Vertreter der disubstituierten Diarylsilane werden aus Dichlordiphenylsilanen und den Natriumsalzen entsprechender Alkan - und Alkensäuren sowie durch Umsetzung mit Peptidketten erhalten. Die Umsetzung erfolgt in stöchiometrischen Anteilen in Ether oder einem anderen Lösungsmittel bei einer Temperatur von ca. 40 °C unter Rühren. Anschließend wird ca. 4 Stunden bei dieser Temperatur nachgerührt. Das ausfallende Produkt wird mit Wasser versetzt und dreimal mit je 10 ml Ether oder einem anderen Lösungsmittel/Lösungsmittelgemisch extrahiert. Die vereinigten Etherextrakte werden getrocknet und im Vakuum eingeengt.

Die Verbindungen der Formel I bilden beim Übergang aus einer organischen Phase in eine wäßrige Phase geordnete Strukturen aus, die in Analogie zu den Liposomen aus Phospholipiden als Siosomen bezeichnet werden. Somit eignen sich die langkettigen Di(acyloxy)dialkylsilane, Di(acyloxy)diarylsilane, Di(acyloxy)-dialkoxysilane und Tetra(acyloxy)silane der Erfindungen zur Herstellung von stabilen Vesikeln auf Nicht-Phospholipid-Basis, den sogenannten Siosomen, die reproduzierbare Eigenschaften aufweisen und gegenüber äußeren Einflüssen, wie Temperatur und Licht, unempfindlich sind. Die Siosomen können als Träger verschiedenartiger Wirkstoffe, wie Pharmazeutika, Enzyme, pflanzliche Wirkstoffe, Kosmetika u.a. eine breite Anwendung finden.

Gegenstand der Erfindung ist deshalb die Verwendung der langkettigen Di(acyloxy)dialkylsilane, Di(acyloxy)diarylsilane, Di(acyloxy)dialkoxysilane und Tetra(acyloxy)silane zur Herstellung von Vesikeln (Siosomen).

In Abhängigkeit von substanzspezifischen Eigenschaften, wie Kettenlänge der Seitenketten der Verbindungen der allgemeinen Formel I, Injektionsgeschwindigkeit, Temperatur und Rührgeschwindigkeit, können Siosomen unterschiedlicher Größe und Struktur (unilamellar, multilamellar und heterolamellar) erhalten werden, die gegenüber Liposomen der Phospholipide den Vorteil besitzen, chemisch stabil zu sein, und die eine definierte Zusammensetzung aufweisen. Siosomen mit heterolamellarer Struktur sind entweder unilamellare Vesikel, die in den größeren multilamellaren Vesikeln eingeschlossen sind oder es handelt sich um multilamellare Vesikel, die in größeren unilamellaren Vesikeln eingeschlossen sind. Ebenso kann bei der Herstellung der Siosomen eine heterogene Mischung von uni-und multilamellaren Vesikeln entstehen. Die Siosomen der Erfindung besitzen gute Einschlußkapazitäten, sind stabil und lassen sich leicht und reproduzierbar herstellen. Es werden Einschlußparameter erreicht, die die Siosomen zu bevorzugten Trägern für verschiedenartige Wirkstoffe machen.

Zur Herstellung der Siosomen aus den langkettigen Di(acyloxy)dialkylsilanen der Erfindung wird beispielsweise eine modifizierte Injektionsmethode benutzt, bei der die wäßrige Phase, die die einzuschließenden Verbindungen enthalten kann, gerührt wird, um das organische Lösungsmittel, das die Siosomen bildenden Verbindungen der allgemeinen Formel I enthält, schneller zu verdampfen. Je nach Löslichkeit kann die einzuschließende Substanz sich entweder in der wäßrigen oder in der organischen Phase befinden.

Die Bereitung der Siosomen-Dispersionen in der oben beschriebenen Form erfolgt vorzugsweise in doppelwandigen, temperierbaren Gefäßen, wobei die Temperatur nahe der Siedetemperatur des verwendeten organischen Lösungsmittels liegt. Von den Herstellungsbedingungen, unter anderem Rührgeschwindigkeit, Injektionsgeschwindigkeit und Temperatur, ist es abhängig, ob unilamellare, und/oder multilamellare, oder/und heterolamellare, kleine oder/und große Siosomen erhalten werden.

Die Verwendung von Siosomen:

1. Die Verwendung von Siosomen als Wirkstoff, Wirkstoff-Prodrug, Wirkstoffe-Pro-Prodrug oder Hilfsstoffe für Humanarzneistoffe, Tierarzneistoffe, Impfstoffe, Enzyme, Coenzyme, Isoenzyme, Vitamine, Hormone, Proteine, Peptide, Kohlenhydrate, Naturstoffe, Kosmetika, Zahnpflegemittel, Haut- und Haarpflegemittel, Hauttalg und Reinigungsmittel, Lebensmittel oder Lebensmittelzusätze, Futtermittel, Konservierungsmittel und andere Zusätze, Riechstoffe, strukturverwandte Riechstoffe wie Pheromone und Aromastoffe, für landwirtschaftliche Zwecke eingesetzte Produkte wie Düngemittel, Biozide wie Pestizide,Herbizide oder Fungizide, Micellenbildner (Detergentien), Desinfektionsmittel, Metallprodukte, Nicht-Metallprodukte, chemische Produkte, Umweltprodukte.

2. Die Verwendung von Siosomen als Pro-Träger und/oder Träger im festen, im flüssigen oder im gasförmigen Zustand als Suspensionen/Dispersionen, als multilamellare Vesikel, unilamellare Vesikel und heterolamellare Vesikel, Reverse-phase Evaporation Vesicles, große einschichtige Vesikel, heterogene Vesikel (multilamellare, unilamellare, heterolamellare, kleine, große).

3. Die Verwendung von Siosomen als Pro-Träger (Pro-Siosomen) mit denen man feste, flüssige oder gasförmige Dispersionen oder Suspensionen aus diesen Silicium-Verbindungen (den Verbindungen im Einzelnen oder als Gemisch mit einem anderen Stoff/Stoffen nach Zugabe und/oder im Beisein von Wasser, wäßrigen Lösungen, Lösungsgemischen, Suspensionen, physiologischen Flüssigkeiten) und Vesikel jeder Art und Größe erhält (multilamellare, unilamellare, heterolamellare, einschichtige, Reverse-phase Evaporation Vesicles).

4. Die Verwendung von Siosomen bei der Vorbereitung, Herstellung und Lagerung von parenteralen Arzneiformen, rektalen und vaginalen Arzneiformen, perkutanen, subkutanen und transdermalen Arzneiformen, Arzneiformen zur Anwendung am Auge, Arzneiformen zur Anwendung in der Nase und dem Ohr, perorale Arzneiformen mit rascher Wirkstofffreisetzung, perorale Arzneiformen mit langsamer Wirkstofffreisetzung, perorale Arzneiformen mit kontrollierter Wirkstofffreisetzung, dentale Applikation oder topische Applikationsformen.

5. Die Verwendung von Siosomen als Bestandteil oder in Mischung mit natürlichen und/oder synthetischen Polymeren aller Art wie Stärke, Stärkehydrolyse- und andere Stärkeabbauprodukte, Polyethylenglycol und andere lösliche, schwerlösliche und unlösliche Polymere.

6. Die Verwendung von Siosomen als Bindemittel, osmotisches Agens, Stabilisator, Antioxidantien, "Penetrationsenhancer", Adjuvantien.

7. Die Verwendung von Siosomen als Pro-Träger, Träger, Bindemittel, Verankerungsmittel für die Vorbereitung, Lagerung und Herstellung von Antigenen, Antigenkonjugaten und Antigenpräparaten, Antikörpern, Antikörperkonjugaten und Antikörperpräparaten, Haptenen, Haptenkonjugaten und Haptenpräparaten, Bakterien, Bakterienkonjugaten und Bakterienpräparaten, Virus, Viruskonjugaten und Viruspräparaten, Heparin, Heparinkonjugaten, Heparinformulierungen und Präparaten, Immunostimulantien, Immunostimulantienkonjugaten und Präparaten, Humanarzneistoffen, Tierarzneistoffen, Impfstoffen, Enzymen, Coenzymen, Isoenzymen, Vitaminen, Hormonen, Proteinen, Peptiden, Kohlenhydraten, Naturstoffen, Kosmetika, Zahnpflegemitteln, Haut- und Haarpflegemitteln, Hauttalg und Reinigungsmitteln, Lebensmitteln, Lebensmittelzusätzen, Futtermitteln, Konservierungsmitteln und anderen Zusätzen, Riechstoffen, strukturverwandten Riechstoffen wie Pheromone und Aromastoffe, für landwirtschaftliche Zwecke eingesetzte Produkte wie Düngemittel, Biozide wie Pestizide, Herbizide oder Fungizide, Micellenbildnern (Detergentien), Desinfektionsmitteln, Metallprodukten, Nicht-Metallprodukten, chemischen Produkten und umweltorientierten Produkten.

8. Die Verwendung von Siosomen bei der Vorbereitung, Herstellung und Lagerung von Diagnostika, Kontrastmitteln und Radiotracern für die Krebsdiagnostik.

9. Die Verwendung von Siosomen bei der Vorbereitung, Herstellung und Lagerung von Kapseln, Microkapseln, Microhohlkugeln, Membranen, Membran-controlled Systems, Hydrogelen, Minipumps für Implantationen, Matrizes.

10. Die Verwendung von Siosomen bei der Vorbereitung, Erstellung und Herstellung von Mitteln für gentechnologische Methoden und Verfahren, analytische Methoden und Verfahren, immunologische Methoden und Verfahren, für in-vivo-Untersuchungen und für in-vitro-Untersuchungen.

11. Die Verwendung zur Herstellung von pulverförmigen Mischungen zur Herstellung von Siosomen für pharmazeutische, medizinische, diagnostische, kosmetische, chemische, umweltorientierte und/oder technische Zwecke.

12. Die Verwendung zur Herstellung von Controlled Release Systems für Arzneimittel, Kosmetika, Pflanzenwirkstoffe, landwirtschaftliche Zwecke eingesetzte Produkte (Düngemittel, Biozide wie Pestizide, Herbizide, Fungizide).

13. Die Verwendung von Siosomen bei der Vorbereitung, Herstellung und Lagerung von Implantierungsmaterialien und Prothesen.

Die Erfindung soll nachstehend an sieben Ausführungsbeispielen näher erläutert werden:

**A. Beispiele für die Herstellung von langkettigen Di(acyloxy)dialkylsilanen, Di(acyloxy)diarylsilanen, Di(acyloxy)dialkoxysilanen und Tetra(acyloxy)silanen:**

<u>Beispiel 1:</u>

Herstellung von Di(decanoyloxy)dimethylsilan

0.012 mol Dimethyldichlorsilan werden in 50 ml wasserfreiem Ether aufgenommen und unter Rühren mit 0.02 mol Natriumdecanoat bei 40 °C versetzt. Um die Ausbeute zu erhöhen, wird ein Überschuß an Dimethyldichlorsilan zugegeben. Anschließend wird ca. drei Stunden bei 40 °C weitergerührt. Zur Hydrolyse des überschüssigen Dimethyldichlorsilans wird mit Wasser versetzt und 3 bis 5 mal mit ca. je 10 ml Ether extrahiert. Die vereinigten Etherextrakte werden über wasserfreiem Natriumsulfat getrocknet und nach Abfiltrieren im Vakuum eingeengt. Das zurückbleibende Di(decanoyloxy)dimethylsilan wird aus Heptan umkristallisiert.

| Summenformel: $C_{22}H_{44}O_4Si$ | |
|---|---|
| Molmasse: | 400.4 |
| Schmelzpunkt: | 32-33 °C |
| Ausbeute: | 92 % |

Beispiel 2:
Herstellung von Di(octadecanoyloxy)dimethylsilan
0.012 mol Dimethyldichlorsilan werden in 50 ml wasserfreiem Ether aufgenommen und unter Rühren mit 0.02 mol Natriumoctadecanoat bei 40 °C versetzt. Anschließend wird wiederum ca. drei Stunden bei dieser Temperatur nachgerührt. Nach der Zersetzung des überschüssigen Dimethyldichlorsilans mit Wasser, wird wie bei Beispiel 1 aufgearbeitet. Aus Heptan wird umkristallisiert.

| Summenformel: $C_{38}H_{76}O_4Si$ | |
|---|---|
| Molmasse: | 624.7 |
| Schmelzpunkt: | 62-64 °C |
| Ausbeute: | 68 % |

Beispiel 3:
Herstellung von Di(octanoyloxy)diphenylsilan:
0.01 mol Dichlordiphenylsilan werden in 50 ml wasserfreiem Ether aufge nommen und unter Rühren mit 0.02 mol Natriumoctanoat bei 40 °C versetzt. Es wird ca. 5 Stunden nachgerührt, anschließend mit Wasser versetzt und mit dreimal je 10 ml Ether extrahiert. Die vereinigten Extrakte werden über Natriumsulfat getrocknet und im Vakuum eingeengt.

| Summenformel: $C_{28}H_{40}O_4Si$ | |
|---|---|
| Molmasse: | 468.4 |
| Schmelzpunkt: | 88-90 °C |
| Ausbeute: | 88 % |

Folgende Substanzen wurden nach den aufgeführten Beispielen synthetisiert:

- Di(decanoyloxy)diphenylsilan:

| Summenformel: $C_{32}H_{48}O_4Si$ | |
|---|---|
| Molmasse: | 524.4 |
| Schmelzpunkt: | 90-92 °C |
| Ausbeute: | 88 % |

- Di(dodecanoyloxy)diphenylsilan:

| Summenformel: $C_{36}H_{56}O_4Si$ | |
|---|---|
| Molmasse: | 580.5 |
| Schmelzpunkt: | 92-95 °C |
| Ausbeute: | 86 % |

- Di(tetradecanoyloxy)diphenylsilan:

| Summenformel: $C_{40}H_{64}O_4Si$ | |
|---|---|
| Molmasse: | 636.6 |
| Schmelzpunkt: | 95-98 °C |
| Ausbeute: | 80 % |

- Di(hexadecanoyloxy)diphenylsilan:

| Summenformel: $C_{44}H_{72}O_4Si$ | |
|---|---|
| Molmasse: | 692.6 |
| Schmelzpunkt: | 98-101 °C |
| Ausbeute: | 82 % |

- Di(octadecanoyloxy)diphenylsilan:

| Summenformel: $C_{48}H_{80}O_4Si$ | |
|---|---|
| Molmasse: | 748.7 |
| Schmelzpunkt: | 101-104 °C |
| Ausbeute: | 80 % |

- Di(octanoyloxy)dimethylsilan:

| Summenformel: $C_{18}H_{36}O_4Si$ | |
|---|---|
| Molmasse: | 344.4 |
| Schmelzpunkt: | < 25 °C |
| Ausbeute: | 82 % |

- Di(dodecanoyloxy)dimethylsilan:

| Summenformel: $C_{26}H_{52}O_4Si$ | |
|---|---|
| Molmasse: | 456.5 |
| Schmelzpunkt: | 38-40 °C |
| Ausbeute: | 88 % |

- Di(tetradecanoyloxy)dimethylsilan:

| Summenformel: $C_{30}H_{60}O_4Si$ | |
|---|---|
| Molmasse: | 512.6 |
| Schmelzpunkt: | 49-51 °C |
| Ausbeute: | 81 % |

- Di(hexadecanoyloxy)dimethylsilan:

| Summenformel: $C_{34}H_{68}O_4Si$ | |
|---|---|
| Molmasse: | 568.6 |
| Schmelzpunkt: | 56-58 °C |
| Ausbeute: | 75 % |

**B. Beispiele für die Herstellung von Siosomen**

Beispiel 4:

Darstellung von Siosomen aus Di(decanoyloxy)dimethylsilan

10 µmol Di(decanoyloxy)dimethylsilan werden in 2 ml Ethanol gelöst und über 4 Stunden langsam dosierend einer die einzuschließenden Verbindungen enthaltenden wäßrigen Phase (Volumen 2 ml), die auf 80 °C temperiert wurde, zugesetzt. Die einzuschließende Verbindung kann auch, entsprechend ihrer Löslichkeit, Bestandteil der organischen Phase sein. Die Injektion erfolgt dann in reines Wasser. Die Rührgeschwindigkeit beträgt hier 2000 ± 200 U/min.

Die eingeschlossenen Verbindungen werden beispielsweise durch Dialyse, Gelfiltration oder durch Zentrifugation von den nichteingeschlossenen Verbindungen abgetrennt und der Inhalt der Siosomen bestimmt. Die so hergestellten Siosomen besaßen eine einheitliche Größe von 300 bis 500 nm und sind offenbar alle unilamellar (Abbildung 1, elektronenmikroskopische Gefrierbruchpräparation von Siosomen aus Di(decanoyloxy)dimethylsilan). Die Stabilität der Siosomen war gut.

Beispiel 5:

Darstellung von Siosomen aus Di(octadecanoyloxy)dimethylsilan

Die Siosomen wurden unter den Bedingungen des Beispieles 1 bereitet. Die elektronenmikroskopische Gefriebruchpräparation (Abbildung 2) zeigt große Siosomen bis zu 2 µm, die meist Mehrschichtstrukturen (multilamellar) aufweisen.

Folgende Siosomen wurden außerdem nach der beschriebenen Methode hergestellt:

- Di(octanoyloxy)dimethylsilan
  weitgehend unilamellar
  einheitliche Größe 200 nm
- Di(dodecanoyloxy)dimethylsilan
  multilamellar
  unterschiedliche Größen bis zu 1.4 µm
- Di(tetradecanoyloxy)dimethylsilan
  multilamellar
  unterschiedliche Größen bis zu 1.5 µm
- Di(hexadecanoyloxy)dimethylsilan
  multilamellar
  unterschiedliche Größen bis zu 1.5 µm

Mit steigender Anzahl von Kohlenstoffatomen in der Seitenkette (von C=8 bis C=18) nimmt die Größe der Siosomen zu (200 nm bis 2 µm). Während bei Di(octanoyloxy)dimethylsilan und bei Di(decanoyloxy)dimethylsilan weitestgehend unilamellare Siosomen einheitlicher Größe entstehen (siehe auch Abb.1), sind bei den anderen erfindungsgemäß hergestellten Siosomen (Anzahl der Kohlenstoffatome in der Seitenkette C = 12 bis C = 18) multilamellare Strukturen vorherrschend (siehe auch Abb.2). Diese Siosomen sind bis zu 2 µm groß und weisen hohe Einschlußkapazitäten auf. Siosomen aus Di(decanoyloxy)dimethylsilan besitzen hohe Stabilität (über mehrere Wochen).

**C. Beispiele für den Einschluß von Wirkstoffen in Siosomen**

Beispiel 6:

Einschluß von Insulin in Di(octadecanoyloxy)dimethylsilan

10 µmol Di(octadecanoyloxy)dimethylsilan werden in 1 ml Ethanol gelöst über 3 Stunden langsam dosierend einer Insulin enthaltenden wäßrigen Phase (Konzentration 10 µg/ml, Volumen 3 ml), die auf 50 °C temperiert und bei 2000 ± 200 U/min gerührt wurde, zugesetzt. Das eingeschlossene Insulin ist durch einstündige Dialyse von dem nicht eingeschlossenen Insulin abgetrennt und anschließend der Insulingehalt mittels einer RIA-Nachweismethode bestimmt worden.

Dabei wurden Einschlußraten von 73,3 - 85,7% eingeschlossenes Insulin ermittelt.

In analoger Weise wurden folgende Vesikelbildner zur Siosomenpräparation eingesetzt und auf ihren Insulineinschluß untersucht. Folgende Einschlußraten wurden ermittelt:

| Silanverbindung | eingeschlossenes Insulin in % |
|---|---|
| Di(tetradecanoyloxy)dimethylsilan | 53,9 - 71,6 |
| Di(octadecanoyloxy)diethylsilan | 42,3 - 66,0 |
| Di(tetradecanoyloxy)diethylsilan | 63,0 - 71,9 |
| Di(octadecanoyloxy)diphenylsilan | 63,8 - 84,4 |
| Di(tetradecanoyloxy)diphenylsilan | 60,1 - 68,7 |

Beispiel 7:
Einschluß von Salicylsäure in Di(decanoyloxy)dimethylsilan
10 µmol Di(decanoyloxy)dimethylsilan werden in 1 ml Ethanol gelöst und über 3 Stunden langsam dosierend einer Salicylsäure enthaltenden wäßrigen Phase (Konzentration 5 µmol/ml, Volumen 2 ml), die auf 60 °C temperiert und bei 2000 ± 200 U/min gerührt wurde, zugesetzt. Die Vesikel mit der eingeschlossenen Salicylsäure sind durch dreistündige Dialyse von der nicht eingeschlossenen Salicylsäure abgetrennt und anschließend nach Zerstörung der Vesikel mit Ethanol die freigesetzte Salicylsäure UV-spektrometrisch vermessen worden.
Dabei wurden Einschlußraten von 53,0 - 68,0% Salicylsäure ermittelt.
In analoger Weise wurde das Di(tetradecanoyloxy)dimethylsilan zur Siosomenbildung eingesetzt.
Folgende Einschlußraten wurden ermittelt:
68,8 - 79,7% eingeschlossene Salicylsäure.

**Patentansprüche**

**1.** Langkettige Di(acyloxy)diarylsilane, Di(acyloxy)dialkoxysilane und Tetra(acyloxy)silane, der allgemeinen Formel I

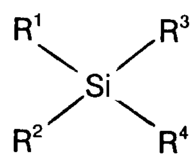

(I)

in der $R^1$ und $R^2$, die gleich oder verschieden sein können, jeweils einen Acyloxyrest der Formel R-COO- oder einen Peptidrest der nachstehenden Formel

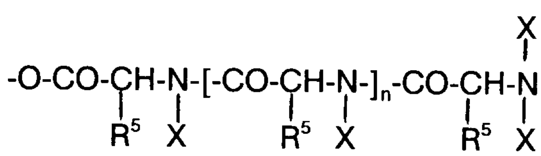

bedeuten, wobei R einen unverzweigten oder verzweigten Alkyl-, Alkenyl- oder Alkinylrest mit 5 bis 29 C-Atomen bedeutet, die durch ein bis drei Halogenatome, Alkoxyreste mit 1 bis 18 C-Atomen oder Aminogruppen substituiert sein können, die Reste $R^5$, die gleich oder verschieden sein können, den nach der Entfernung der Gruppe

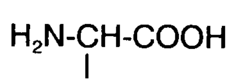

von einer in der Natur vorkommenden Aminosäure verbleibenden Rest darstellt, die Reste X, die gleich oder verschieden sind, ein Wasserstoffatom oder eine in der Peptidchemie übliche Aminoschutzgruppe darstellen und n eine ganze Zahl von 1 bis 12 bedeutet, und $R^3$ und $R^4$, die gleich oder verschieden sind, jeweils eine Arylgruppe, beispielsweise Phenylgruppe, oder den nach Entfernung eines Wasserstoffatoms verbleibenden Rest eines Monosaccharids, Disaccharids, Aminozuckers oder einer Hydroxycarbonsäure, oder Alkoxyreste mit 1 bis 5 C-Atomen oder Acylreste einer in der Natur vorkommenden Aminosäure mit freier oder geschützter Aminogruppe oder Di-, Tri-, oder Tetrapeptide aus in der Natur vorkommenden Aminosäuren mit freien oder geschützten Aminogruppen oder entsprechend $R^1$, $R^2$, wobei R einen unverzweigten oder verzweigten Alkyl-, Alkenyl- oder Alkinylrest darstellen.

**2.** Verbindungen nach Anspruch 1 der allgemeinen Formel I, in der $R^1$ und $R^2$ jeweils einen Rest der Formel R-COO- darstellen, wobei R einen Alkylrest mit 5 bis 17 C-Atomen oder einen ein- oder mehrfach ungesättigten Alkenylrest mit 5 bis 17 C-Atomen, oder einen ein- oder mehrfach ungesättigten Alkinylrest mit 5 bis 17 C-Atomen bedeutet.

**3.** Verbindungen nach Anspruch 1 der allgemeinen Formel I, in der $R^1$ und $R^2$ jeweils eine Polypeptidkette der Formel

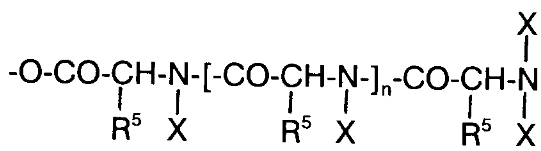

bedeuten, in der n einen Wert von 1 bis 12 aufweist und die Reste $R^5$ und X die im Anspruch 1 angegebene Bedeutung haben.

**4.** Verbindungen nach Anspruch 1 oder 3 der allgemeinen Formel I, in der die Reste $R^5$ die gleiche Bedeutung haben.

**5.** Verbindungen nach Anspruch 1 oder 3 der allgemeinen Formel I, in der die Reste $R^5$ verschiedene Bedeutungen haben.

**6.** Verbindungen nach Anspruch 1 bis 5 der allgemeinen Formel I, in denen die Reste $R^1$ und $R^2$ sowie die Reste $R^3$ und $R^4$ jeweils die gleiche Bedeutung haben.

**7.** Verwendung der Verbindungen nach den Ansprüchen 1 bis 6 zur Herstellung von Vesikeln.

**8.** Verwendung der langkettigen Di(acyloxy)dialkylsilane der allgemeinen Formel I, in der $R^1$ und $R^2$, die in den Ansprüchen 1 bis 6 angegebene Bedeutung haben, $R^3$ und $R^4$, die gleich oder verschieden sein können, jeweils einen Alkylrest mit 1 bis 6 C-Atomen darstellen, zur Herstellung von Vesikeln.

**9.** Vesikel (Siosom), bestehend aus mindestens einer Schicht von langkettigen Di(acyloxy)-dialkylsilanen,Di(acyloxy)diarylsilanen,Di(acyloxy)dialkoxysilanen und Tetra(acyloxy)silanen der allgemeinen Formel I gemäß einem der Ansprüche 1 bis 8.

**10.** Vesikel (Siosom) nach Anspruch 9, dadurch gekennzeichnet, daß sie unilamellar sind.

**11.** Vesikel (Siosom) nach Anspruch 9, dadurch gekennzeichnet, daß sie multilamellar sind.

**12.** Vesikel (Siosom) nach Anspruch 9, dadurch gekennzeichnet, daß sie heterolamellar sind.

**13.** Verfahren zur Herstellung der Vesikel (Siosomen) dadurch gekennzeichnet, daß man ein langkettiges Di(acyloxy)dialkylsilan, Di(acyloxy)diarylsilan, Di(acyloxy)dialkoxysilan und Tetra(acyloxy)silan der allgemeinen Formel I in einem Lösungsmittel, einem organischen Lösungsmittel oder Lösungsmittelgemisch löst, die Lösung in eine wäßrige Phase oder organische Phase oder Mischphase einbringt und das Lösungsmittel entfernt.

**14.** Verwendung der Siosomen nach den Ansprüchen 9 bis 12 als Wirkstoff, Wirkstoff-Prodrug, Wirkstoffe-Pro-Prodrug oder Hilfsstoffe für Humanarzneistoffe, Tierarzneistoffe, Impfstoffe, Enzyme, Coenzyme,

Isoenzyme, Vitamine, Hormone, Proteine, Peptide, Kohlenhydrate, Naturstoffe, Kosmetika, Zahnpflegemittel, Haut- und Haarpflegemittel, Hauttalg und Reinigungsmittel, Lebensmittel oder Lebensmittelzusätze, Futtermittel, Konservierungsmittel und andere Zusätze, Riechstoffe, strukturverwandte Riechstoffe wie Pheromone und Aromastoffe, für landwirtschaftliche Zwecke eingesetzte Produkte wie Düngemittel, Biozide wie Pestizide,Herbizide oder Fungizide, Micellenbildner (Detergentien), Desinfektionsmittel, Metallprodukte, Nicht-Metallprodukte, chemische Produkte, Umweltprodukte.

**15.** Verwendung der Siosomen nach den Ansprüchen 9 bis 12 als Pro-Träger und/oder Träger im festen, im flüssigen oder im gasförmigen Zustand als Suspensionen/Dispersionen, als multilamellare Vesikel (Siosomen), unilamellare Vesikel (Siosomen) und heterolamellare Vesikel (Siosome), Reversephase Evaporation Vesicles (Siosomen), große einschichtige Vesikel (Siosomen), heterogene Vesikel (Siosomen) (multilamellare, unilamellare, heterolamellare, kleine, große).

**16.** Verwendung der Siosomen nach den Ansprüchen 9 bis 12 als Pro-Träger (Pro-Siosomen) mit denen man feste, flüssige oder gasförmige Dispersionen oder Suspensionen aus diesen Silicium-Verbindungen (den Verbindungen im Einzelnen oder als Gemisch mit einem anderen Stoff/Stoffen nach Zugabe und/oder im Beisein von Wasser, wäßrigen Lösungen, Lösungsgemischen, Suspensionen, physiologischen Flüssigkeiten) und Vesikel (Siosomen) jeder Art und Größe erhält (multilamellare, unilamellare, heterolamellare, einschichtige, Reverse-phase Evaporation Vesicles).

**17.** Verwendung der Siosomen nach den Ansprüchen 9 bis 12 bei der Vorbereitung, Herstellung und Lagerung von parenteralen Arzneiformen, rektalen und vaginalen Arzneiformen, perkutanen, subkutanen und transdermalen Arzneiformen, Arzneiformen zur Anwendung am Auge, Arzneiformen zur Anwendung in der Nase und dem Ohr, perorale Arzneiformen mit rascher Wirkstofffreisetzung, perorale Arzneiformen mit langsamer Wirkstofffreisetzung, perorale Arzneiformen mit kontrollierter Wirkstofffreisetzung, dentale Applikation oder topische Applikationsformen.

**18.** Verwendung der Siosomen nach den Ansprüchen 9 bis 12 als Bestandteil oder in Mischung mit natürlichen und/oder synthetischen Polymeren aller Art wie Stärke, Stärkehydrolyse- und andere Stärkeabbauprodukte, Polyethylenglycol und andere lösliche, schwerlösliche und unlösliche Polymere.

**19.** Verwendung der Siosomen nach den Ansprüchen 9 bis 12 als Bindemittel, osmotisches Agens, Stabilisator, Antioxidantien, "Penetrationsenhancer", Adjuvantien.

**20.** Verwendung der Siosomen nach den Ansprüchen 9 bis 12 als Pro-Träger, Träger, Bindemittel, Verankerungsmittel für die Vorbereitung, Lagerung und Herstellung von Antigenen, Antigenkonjugaten und Antigenpräparaten, Antikörpern, Antikörperkonjugaten und Antikörperpräparaten, Haptenen, Haptenkonjugaten und Haptenpräparaten, Bakterien, Bakterienkonjugaten und Bakterienpräparaten, Virus, Viruskonjugaten und Viruspräparaten, Heparin, Heparinkonjugaten, Heparinformulierungen und Präparaten, Immunostimulantien, Immunostimulantienkonjugaten und Präparaten, Humanarzneistoffen, Tierarzneistoffen, Impfstoffen, Enzymen, Coenzymen, Isoenzymen, Vitaminen, Hormonen, Proteinen, Peptiden, Kohlenhydraten, Naturstoffen, Kosmetika, Zahnpflegemitteln, Haut- und Haarpflegemitteln, Hauttalg und Reinigungsmitteln, Lebensmitteln, Lebensmittelzusätzen, Futtermitteln, Konservierungsmitteln und anderen Zusätzen, Riechstoffen, strukturverwandten Riechstoffen wie Pheromone und Aromastoffe, für landwirtschaftliche Zwecke eingesetzte Produkte wie Düngemittel, Biozide wie Pestizide, Herbizide oder Fungizide, Micellenbildnern (Detergentien), Desinfektionsmitteln, Metallprodukten, Nicht-Metallprodukten, chemischen Produkten und umweltorientierten Produkten.

**21.** Verwendung der Siosomen nach den Ansprüchen 9 bis 12 bei der Vorbereitung, Herstellung und Lagerung von Diagnostika, Kontrastmitteln und Radiotracern für die Krebsdiagnostik.

**22.** Verwendung der Siosomen nach den Ansprüchen 9 bis 12 bei der Vorbereitung, Herstellung und Lagerung von Kapseln, Microkapseln, Microhohlkugeln, Membranen, Membran-controlled Systems, Hydrogelen, Minipumps für Implantationen, Matrizes.

**23.** Verwendung der Siosomen nach den Ansprüchen 9 bis 12 bei der Vorbereitung, Erstellung und Herstellung von Mitteln für gentechnologische Methoden und Verfahren, analytische Methoden und Verfahren, immunologische Methoden und Verfahren, für in-vivo-Untersuchungen und für in-vitro-Untersuchungen.

**24.** Verwendung der Siosomen nach den Ansprüchen 9 bis 12 zur Herstellung von pulverförmigen Mischungen zur Herstellung von Siosomen für pharmazeutische, medizinische, diagnostische, kosmetische, chemische, umweltorientierte und/oder technische Zwecke.

**25.** Verwendung der Siosomen nach den Ansprüchen 9 bis 12 zur Herstellung - von Controlled Release Systems für Arzneimittel, Kosmetika, Pflanzenwirkstoffe, landwirtschaftliche Zwecke eingesetzte Produkte (Düngemittel, Biozide wie Pestizide, Herbizide, Fungizide).

**26.** Verwendung der Siosomen nach den Ansprüchen 9 bis 12 bei der Vorbereitung, Herstellung und Lagerung von Implantierungsmaterialien und Prothesen.

## Claims

**1.** Long-chain, di(acyloxy)diarylsilanes, di(acyloxy)dialkoxysilanes and tetra(acyloxy)silanes composed according to the general Formula I

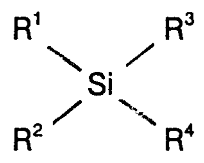

(I)

$R^1$ and $R^2$, which can be the same or different, each represent an acyloxy residue with the formula R - COO - or a peptide residue of the formula

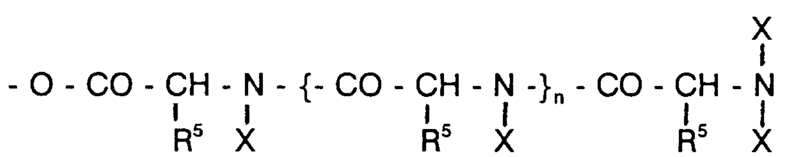

with R representing an unbranched or branched alkyl, alkenyl or alkinyl residue with 5 to 29 C-atoms which can be substituted by one to three halogen atoms, alkoxy residues with 1 to 18 C-atoms or amino groups. The residues $R^5$, which can be the same or different, represent the residue remaining after the removal of the group

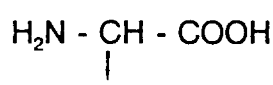

from an amino acid occurring in nature. The residues X, which may be the same or different, represent a hydrogen atom or an amino-protective group usually occurring in peptide chemistry. n represents a whole number from 1 to 12. $R^3$ and $R^4$, which can be the same or different, each represent an aryl group, such as a phenyl group for example, or the residue remaining after the removal of a hydrogen atom from a monosaccharide, disaccharide, amino sugar or a hydroxy carbon acid, or alkoxy residues with 1 - 5 C-atoms, or acyl residues of an amino acid occurring in nature with a free or protected amino group, or a dipeptide, tripeptide or tetrapeptide residue of an amino acid occurring in nature with free or protected amino groups, or they have the meaning $R^1$ and $R^2$, with R representing an unbranched or branched alkyl, akenyl or alkinyl residue.

**2.** Compounds according to Claim 1, composed according to the general Formula I, in which $R^1$ and $R^2$ each represent a residue of the formula R - COO -, with R representing an alkyl residue with 5 to 17 C-atoms or a monounsaturated or polyunsaturated alkenyl residue with 5 to 17 C-atoms, or a monounsaturated or polyunsaturated alkinyl residue with 5 to 17 C-atoms

**3.** Compounds according to Claim 1, composed according to the general Formula I, in which $R^1$ and $R^2$ each represent a polypeptide chain with the formula

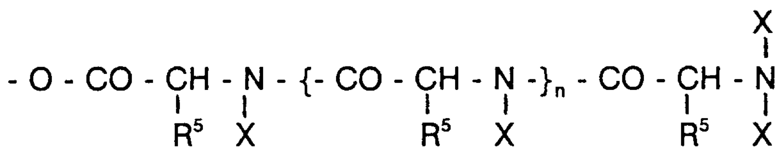

with n having a value of 1 to 12, and the residues $R^5$ and X having the meanings given in Claim 1.

**4.** Compounds according to Claims 1 or 3, composed according to the general Formula I, in which the residues $R^5$ have the same meaning.

**5.** Compounds according to Claims 1 or 3, composed according to the general Formula I, in which the residues $R^5$ have different meanings.

**6.** Compounds according to Claims 1 to 5, composed according to the general Formula 1, in which the residues $R^1$ and $R^2$ and the residues $R^3$ and $R^4$, respectively, have the same meaning.

**7.** Use of the compounds according to Claims 1 to 6 for the preparation of vesicles.

**8.** Use of long-chain di(acyloxy)dialkylsilanes composed according to the general Formula I, in which the residues $R^1$ and $R^2$ according to Claims 1 to 6, and the residues $R^3$ and $R^4$ have the same or different meanings, each represent an alkyl residue with 1 to 6 C-atoms, for the preparation of vesicles.

**9.** Vesicles (Siosomes) consisting of at least one layer of long-chain di(acyloxy)dialkylsilanes, di(acyloxy)-diarylsilanes, di(acyloxy)dialkoxysilanes and tetra(acyloxy)silanes composed according to the general Formula I, according to one of the Claims 1 to 8.

**10.** Vesicles (Siosomes) according to Claim 9, distinguished by being unilamellar.

**11.** Vesicles (Siosomes) according to Claim 9, distinguished by being multilamellar.

**12.** Vesicles (Siosomes) according to Claim 9, distinguished by being heterolamellar.

**13.** Methods of vesicle (Siosome) preparation, distinguished by the fact that a long-chain di(acyloxy)-dialkylsilane, di(acyloxy)diarylsilane, di(acyloxy)dialkoxysilane and tetra(acyloxy)silane composed according to the general Formula I is dissolved in a solvent, an organic solvent or solvent mixture, the solution is added to an aqueous phase or organic phase or mixed phase, then the solvent is removed.

**14.** Use of the Siosomes prepared according to Claims 10 to 13 as active substance, active substance-prodrug, active substance-pro-prodrug or inactive substances for drugs for human use, veterinary drugs, vaccines, enzymes, coenzymes, isoenzymes, vitamins, hormones, proteins, peptides, carbohydrates, natural substances, cosmetics, dental care products, skin and hair care products, sebum and detergents, foodstuffs or food additives, animal feeds, preservatives and other additives, perfumes, perfumes related in structure such as pheromones and flavorings, products used in agriculture such as fertilizers, biocides such as pesticides, herbicides or fungicides, micelle formers (detergents), disinfectants, metal products, non-metal products, chemical products, environmental products.

**15.** Use of the Siosomes prepared according to Claims 9 to 12 as pro-vehicle and/or vehicle in the solid, liquid or gaseous state as suspensions/dispersions, as multilamellar vesicles (Siosomes), unilamellar vesicles (Siosomes) and heterolamellar vesicles (Siosomes), reverse phase evaporation vesicles (Siosomes), large monolayer vesicles (Siosomes), heterogeneous vesicles (Siosomes) (multilamellar, unilamellar, heterolamellar, small, large).

**16.** Use of the Siosomes prepared according to Claims 9 to 12 as pro-vehicles (pro-Siosomes) to produce solid, liquid or gaseous dispersions or suspensions from these silicon compounds (the individual compounds or as a mixture with (an)other substance(s) after addition and/or in the presence of water, aqueous solutions, solution mixtures, suspensions, physiological liquids) and vesicles (Siosomes) of every type and size (multilamellar, unilamellar, heterolamellar, monolayer, reverse-phase evaporation vesicles).

**17.** Use of the Siosomes prepared according to Claims 9 to 12 in the preparation, manufacture and storage of parenteral dosage forms, rectal and vaginal dosage forms, percutaneous, subcutaneous and transdermal dosage forms, formulations for ophthalmic, nasal and aural application, immediate release oral dosage forms, sustained release oral dosage forms, controlled release oral formulations, for dental application or topical dosage forms.

**18.** Use of the Siosomes prepared according to Claims 9 to 12 as an ingredient of or mixed with all kinds of natural and/or synthetic polymers such as starch, starch hydrolysis and other starch degradation products, polyethylene glycol and other soluble, sparingly soluble and insoluble polymers.

**19.** Use of the Siosomes prepared according to Claims 9 to 12 as a binding agent, osmotic agent, stabilizer, antioxidants, penetration enhancers, adjuvants.

**20.** Use of the Siosomes prepared according to Claims 9 to 12 as pro-vehicle, vehicle, binding agent, anchoring agent for the preparation, storage and manufacture of antigens, antigen conjugates and antigen preparations, antibodies, antibody conjugates and antibody preparations, haptenes, haptene conjugates and haptene preparations, bacteria, bacterial conjugates and bacterial preparations, viruses, virus conjugates and virus preparations, heparin, heparin conjugates, heparin formulations and preparations, immunostimulants, immunostimulant conjugates and preparations, drugs for human use, veterinary drugs, vaccines, enzymes, coenzymes, isoenzymes, vitamins, hormones, proteins, peptides, carbohydrates, natural substances, cosmetics, dental care products, skin and hair care products, sebum and detergents, foodstuffs, food additives, animal feeds, preservatives and other additives, perfumes, perfumes related in structure such as pheromones and flavorings, products used in agriculture such as fertilizers, biocides such as pesticides, herbicides or fungicides, micelle formers (detergents), disinfectants, metal products, non-metal products, chemical products and environment-oriented products.

**21.** Use of the Sisomes prepared according to Claims 9 to 12 in the preparation, manufacture and storage of diagnostic agents, contrast media and radiotracers for cancer diagnosis.

**22.** Use of the Siosomes prepared according to Claims 9 to 12 in the preparation, manufacture and storage of capsules, microcapsules, microscopic hollow globules, membranes, membrane-controlled systems, hydrogels, minipumps for implants, matrices.

**23.** Use of the Siosomes prepared according to Claims 9 to 12 in the preparation, development and production of agents for genetic engineering methods and procedures, analytical methods and procedures, immunological methods and procedures, for in vivo evaluations and in vitro testing.

**24.** Use of the Siosomes prepared according to Claims 9 to 12 for the manufacture of powder mixes for Siosome production for pharmaceutical, medical, diagnostic, cosmetic, chemical, environment-oriented and/or technical purposes.

**25.** Use of the Siosomes prepared according to Claims 9 to 12 for the manufacture of controlled release systems for drugs, cosmetics, phytodrugs, products used in agriculture (fertilizers, biocides such as pesticides, herbicides, fungicides).

**26.** Use of the Siosomes prepared according to Claims 9 to 12 in the preparation, manufacture and storage of implant materials and prosthetic devices.

## Revendications

1. Di(acyloxy)diarylsilanes, di(acyloxy)dialkoxysilanes et tétra(acyloxy)silanes à longue chaîne, de formule générale

```
 R1      R3
   \    /
     Si             (I)
   /    \
 R2      R4
```

dans lesquels R1 et R2, qui peuvent être semblables ou différents, représentent respectivement un reste acyloxy de formule R-COO- ou une chaîne peptidique possédant la structure suivante:

```
                                           X
                                           |
-O-CO-CH-N-[-CO-CH-N-]n-CO-CH-N
      |  |       |  |      |  |
      R5 X       R5 X      R5 X
```

où R représente un reste alkyle, alcène ou alkine, ramifié ou non ramifié, comprenant de 5 à 29 carbones, qui eux-mêmes peuvent être substitués soit par 1 à 3 halogènes, soit par des restes alkoxy contenant de 1 à 18 carbones, soit par des groupes aminés; où les restes R5, qui peuvent être semblables ou différents, représentent le reste d'un acide aminé naturel résultant de l'élimination du groupe

```
H2N-CH-COOH
     |
```

où les restes X, qui peuvent être semblables ou différents, représentent un atome d'hydrogène ou un groupe amine protecteur fréquent dans la chimie des peptides; où n signifie un nombre entier de 1 à 12; et où R3 et R4, qui peuvent être semblables ou différents, représentent soit un groupe aryl, par exemple phényl, soit après élimination d'un atome d'hydrogène, un reste de mono- ou de disaccharide, de sucre aminé ou d'acide hydroxycarbonique, soit des restes alkoxy comprenant de 1 à 5 carbones, soit des restes acyles d'un acide aminé naturel avec la fonction amine libre ou protégée, soit des di-, tri- ou tétrapeptides d'acides aminés naturels avec les fonctions amines libres ou protégées, soit les mêmes restes que R1 et R2, auquel cas R représente un reste alkyle, alcène ou alkine ramifié ou non ramifié.

2. Composés définis par la revendication 1., de formule générale I, dans laquelle R1 et R2 représentent respectivement un reste de formule R-COO-, où R a la signification d'un reste alkyle comprenant de 5 à 17 carbones, ou bien d'un reste alcène, mono- ou polyinsaturé, comprenant de 5 à 17 carbones, ou encore d'un reste alkine, mono- ou polyinsaturé, comprenant de 5 à 17 carbones.

3. Composés définis par la revendication 1., de formule générale I, dans lesquels R1 et R2 représentent une chaîne polypeptidique de formule

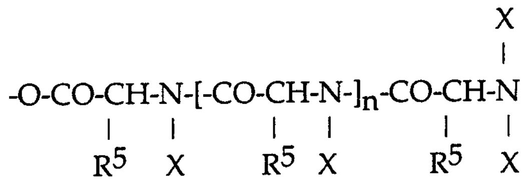

dans laquelle n signifie un nombre de 1 à 12 et les restes R5 et X ont les caractéristiques définies dans la revendication 1.

**4.** Composés définis selon les revendications 1.ou 3., de formule générale I, dans lesquels les restes R5 ont la même signification.

**5.** Composés définis selon les revendications 1. ou 3., de formule générale I, dans lesquels les restes R5 ont des significations différentes.

**6.** Composés définis selon les revendications 1. à 5., de formule générale I, dans lesquels les restes R1 et R2 d'une part, R3 et R4 d'autre part ont respectivement la même signification.

**7.** Utilisation des composés définis selon les revendications 1. à 6., pour la fabrication de vésicules.

**8.** Utilisation des di(acyloxy)dialkylsilanes à longue chaîne, de formule générale I, dans lesquels R1 et R2, qui obéissent à la définition donnée dans les revendications 1. à 6., et dans lesquels R3 et R4, qui peuvent être semblables ou différents, représentent respectivement un reste alkyle comprenant de 1 à 6 carbones, pour la fabrication de vésicules.

**9.** Vésicules (siosomes) formées d'au moins une couche de di(acyloxy)dialkylsilanes, di(acyloxy)-diarylsilanes, di(acyloxy)dialkoxysilanes et tétra(acyloxy)silanes à longue chaîne,de formule générale I, conformément à l'une des revendications 1. à 8.

**10.** Vésicules (siosomes) définies selon la revendication 9., caractérisées par leur nature unilamellaire.

**11.** Vésicules (siosomes) définies selon la revendication 9., caractérisées par leur nature multilamellaire.

**12.** Vésicules (siosomes) définies selon la revendication 9., caractérisées par leur nature hétérolamellaire.

**13.** Procédé de fabrication des vésicules (siosomes) caractérisé par le fait que l'on dissout un di(acyloxy)-dialkylsilane, un di(acyloxy)diarylsilane, un di(acyloxy)dialkoxysilane et un tétra(acyloxy)silane à longue chaîne,de formule générale I, dans un solvant, un solvant organique ou un mélange de solvants, que l'on transfère la solution obtenue dans une phase aqueuse, organique ou dans un mélange des deux, et que l'on élimine le solvant.

**14.** Utilisation des siosomes selon les revendications 9. à 12., comme principe actif, précurseur ou pré-précurseur de principe actif, ou comme excipient de médicaments à destination humaine et animale, vaccins, enzymes, coenzymes, isoenzymes, vitamines, hormones, protéines, peptides, hydrates de carbone, substances naturelles, cosmétiques, produits d'hygiène dentaire, cutanée et capillaire, sébum, produits d'entretien, denrées alimentaires ou additifs, aliments pour animaux, conservateurs et autres additifs, parfums, parfums de structure apparentée tels que phéromones et arômes, produits à usage agricole tels qu'engrais, biocides tels que pesticides, herbicides ou fongicides, détergents, désinfectants, produits métalliques et non métalliques, produits chimiques, produits en rapport avec la protection de l'environnement.

**15.** Utilisation des siosomes selon les revendications 9. à 12., comme précurseurs de supports et/ou comme supports à l'état solide, liquide ou gazeux, comme suspensions/dispersions, comme vésicules multilamellaires (siosomes), unilamellaires (siosomes) et hétérolamellaires (siosomes), Reverse-phase Evaporation Vesicles (siosomes), grosses vésicules formées d'une seule couche (siosomes), vésicules hétérogènes (siosomes) (multilamellaires, unillamellaires, hétérolamellaires, petites, grosses).

**16.** Utilisation des siosomes selon les revendications 9. à 12., comme précurseurs de supports (prosiosomes), avec lesquels on obtient des dispersions ou des suspensions solides, liquides ou gazeuses de ces composés du silicium (composés isolés ou mélangés à un autre/d'autres produit(s) après addition et/ou en présence d'eau, de solutions aqueuses, de mélanges de solutions, de suspensions, de liquides physiologiques) et des vésicules (siosomes) de toute catégorie et de toute taille (multilamellaires, unilamellaires, hétérolamellaires, formées d'une seule couche, Reverse-phase Evaporation Vesicles).

**17.** Utilisation des siosomes selon les revendications 9. à 12., dans la préparation, la fabrication et le stockage de médicaments destinés à la voie parentérale, rectale, vaginale, percutanée, sous-cutanée, intradermique, de médicaments à usage oculaire, nasal, auriculaire, de préparations orales à action rapide, lente, contrôlée, de médicaments à application dentaire ou topique.

**18.** Utilisation des siosomes selon les revendications 9. à 12., dans la composition de (ou en mélange avec des) polymères naturels et/ou synthétiques de toutes sortes tels que l'amidon et ses dérivés hydrolytiques ou autres, le polyéthylène glycol ainsi que d'autres polymères solubles, peu solubles ou insolubles.

**19.** Utilisation des siosomes selon les revendications 9. à 12., comme agglutinants, agents osmotiques, stabilisateurs, antioxydants, "penetrationsenhancers", adjuvants.

**20.** Utilisation des siosomes selon les revendications 9. à 12., comme précurseurs de supports, supports, agglutinants, agents d'ancrage pour la préparation, le stockage et la fabrication d'antigènes, conjugués d'antigènes et préparations antigéniques, anticorps, conjugués d'anticorps et préparations d'anticorps, haptènes, conjugués d'haptènes et préparations d'haptènes, bactéries, conjugués bactériens et préparations bactériennes, virus, conjugués viraux et préparations virales, héparine, conjugués d'héparine, formulations et préparations d'héparine, immunostimulants ainsi que leurs conjugués et préparations, médicaments à destination humaine et animale, vaccins, enzymes, coenzymes, isoenzymes, vitamines, hormones, protéines, peptides, hydrates de carbone, substances naturelles, cosmétiques, produits d'hygiène dentaire, cutanée et capillaire, sébum, produits d'entretien, denrées alimentaires ou additifs, aliments pour animaux, conservateurs et autres additifs, parfums, parfums de structure apparentée tels que phéromones et arômes, produits à usage agricole tels qu'engrais, biocides tels que pesticides, herbicides ou fongicides, détergents, désinfectants, produits métalliques et non métalliques, produits chimiques, produits en rapport avec la protection de l'environnement.

**21.** Utilisation des siosomes selon les revendications 9. à 12., dans la préparation, la fabrication et le stockage de produits diagnostiques, de moyens de contraste, de traceurs radioactifs pour le diagnostic des cancers.

**22.** Utilisation des siosomes selon les revendications 9. à 12., dans la préparation, la fabrication et le stockage de capsules, microcapsules, microsphères creuses, membranes, Membran-controlled Systems, hydrogels, minipompes pour implantations, matrices.

**23.** Utilisation des siosomes selon les revendications 9. à 12., dans la préparation, l'élaboration et la fabrication de produits utilisés dans les méthodes et techniques du génie génétique, dans les méthodes et techniques analytiques, dans les méthodes et techniques immunologiques, ainsi que dans les études in vivo et in vitro.

**24.** Utilisation des siosomes selon les revendications 9. à 12., dans la fabrication de mélanges conditionnés sous forme de poudres, destinés à la fabrication de siosomes à usage pharmaceutique, médical, diagnostique, cosmétique, chimique, écologique et/ou technique.

**25.** Utilisation des siosomes selon les revendications 9. à 12., dans la fabrication de Controlled Release Systems pour préparations médicales, cosmétiques, produits végétaux, produits d'usage agricole (engrais, biocides tels que pesticides, herbicides, fongicides).

**26.** Utilisation des siosomes selon les revendications 9. à 12., dans la préparation, la fabrication et le stockage de matériaux d'implantation et de prothèses.

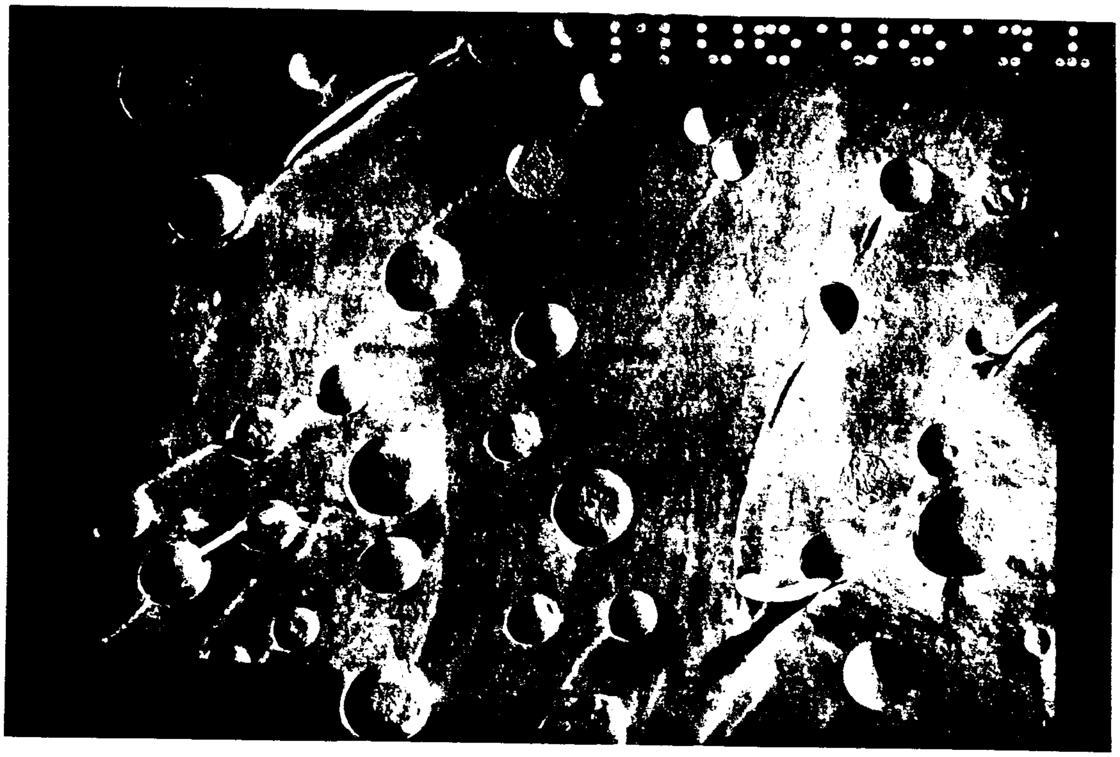

Abbildung 1

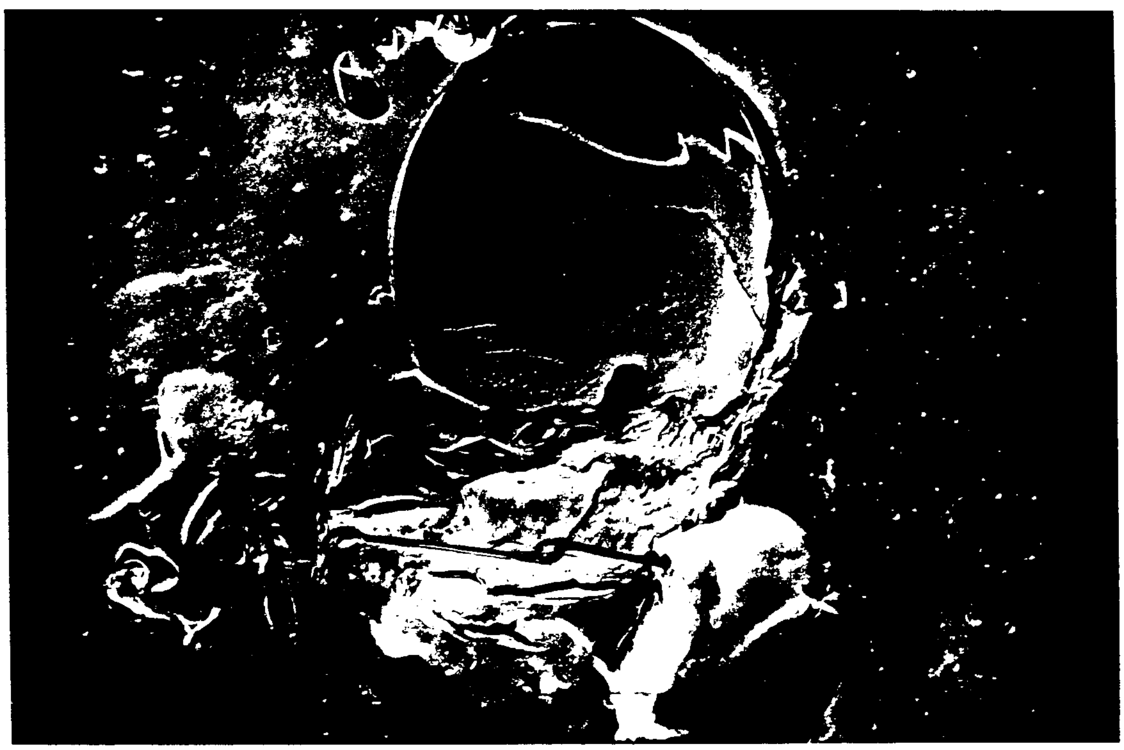

Abbildung 2